# EUROPEAN PATENT SPECIFICATION

(11) **EP 3 528 823 B1**
(45) Date of publication and mention of the grant of the patent: **18.09.2024**
(21) Application number: 17865812.6
(22) Date of filing: 23.10.2017
(51) Int. Cl.: A61K 35/12, A61K 35/17, A61P 35/00, C07K 14/725, C12N 5/00, C12N 5/0783, C07K 14/705

(54) **DETERMINISTIC LATERAL DISPLACEMENT IN THE PREPARATION OF CELLS AND COMPOSITIONS FOR THERAPEUTIC USES**
DETERMINISTISCHE LATERALE VERSCHIEBUNG BEI DER HERSTELLUNG VON ZELLEN UND ZUSAMMENSETZUNGEN FÜR THERAPEUTISCHE VERWENDUNGEN
DÉPLACEMENT LATÉRAL DÉTERMINISTE DANS LA PRÉPARATION DE CELLULES ET DE COMPOSITIONS À USAGES THÉRAPEUTIQUES

(30) Priority: 24.10.2016 US 201662412180 P; 01.09.2017 US 201762553723 P; 03.10.2017 US 201762567553 P
(43) Date of publication of application: 28.08.2019
(73) Proprietor: GPB Scientific, Inc., Richmond, VA 23219 (US); The Trustees of Princeton University, Princeton, NJ 08544 (US); University of Maryland, Baltimore, Baltimore, MD 21201 (US)
(72) Inventor: WARD, Anthony, Rancho Santa Fe CA 90267 (US); CAMPOS-GONZALEZ, Roberto, Carlsbad CA 92009 (US); SKELLEY, Alison, Riverside CA 92508 (US); GANDHI, Khushroo, Palo Alto CA 94306 (US); GRISHAM, Michael, Richmond VA 23221 (US); CIVIN, Curt, Baltimore MD 21230 (US); STURM, James, C., Princeton NJ 08540 (US)
(74) Representative: Maiwald GmbH
(86) International application number: PCT/US2017/057876
(87) International publication number: WO 2018/080997

(56) References cited:
- WO-A1-2015/084257
- WO-A1-2015/162211
- WO-A1-2015/162211
- WO-A1-2015/164745
- WO-A2-2014/145075
- WO-A2-2014/145152
- US-A1- 2004 224 402
- DAVID HOLMES ET AL: "Separation of blood cells with differing deformability using deterministic lateral displacement", INTERFACE FOCUS, vol. 4, no. 6, 6 December 2014 (2014-12-06), GB, pages 20140011, XP055673013, ISSN: 2042-8898, DOI: 10.1098/rsfs.2014.0011
- NAN LI ET AL: "On-Chip Continuous Blood Cell Subtype Separation by Deterministic Lateral Displacement", NANO/MICRO ENGINEERED AND MOLECULAR SYSTEMS, 2007. NEMS '07. 2ND IEEE INTERNATIONAL CONFERENCE ON, IEEE, PISCATAWAY, NJ, USA, 1 January 2007 (2007-01-01), pages 932 - 936, XP031079897, ISBN: 978-1-4244-0610-4
- DAVID W. INGLIS ET AL: "Microfluidic device for label-free measurement of platelet activation", LAB ON A CHIP, vol. 8, no. 6, 1 January 2008 (2008-01-01), pages 925, XP055282905, ISSN: 1473-0197, DOI: 10.1039/b800721g

## Description

### Field of the Invention

The present invention is directed primarily to *in vitro* methods of preparing cells and compositions for therapeutic uses. The methods employ microfluidic devices that use Deterministic Lateral Displacement to separate cells based on size.

### Background of the Invention

Cell therapy, and especially CAR-T cell therapy, has demonstrated extraordinary efficacy in treating B-cell diseases such as B-acute lymphoid leukemia (B-ALL) and B-Cell Lymphomas. As a result, the demand for autologous therapies has increased dramatically and development efforts have broadened to focus on cancers characterized by solid tumors, such as glioblastomas (Vonderheide, et al., Immunol. Rev. 257:7-13 (2014); Fousek, et al., Clin. Cancer Res. 21:3384-3392 (2015); Wang, et al., Mol. Ther. Oncolytics 3: 16015 (2016); Sadelain, et al., Nature 545:423-431 (2017)). Targeted gene editing with CRISPR/Cas-9 in focused populations of autologous cells, such as stem cells, may further fuel demand (Johnson, et al., Cancer Cell Res. 27:38-58 (2017)).

The preparation of cells for personalized therapy is usually a labor-intensive process that relies on procedures adapted from blood banking or protein bioprocessing procedures which are poorly suited for therapeutic applications. Cell losses associated with processing steps are typically substantial (Hokland, etal., Scand. J. Immunol. 11:353-356 (1980); Stroncek, et al., J. Transl. Med. 12:241 (2014)), in part because of processes that use preparations that achieve cell specific separations (Powell, et al., Cytotherapy 11:923-935 (2009); TerumoBCT. ELUTRA Cell Separation System. Manufacturer recommendations for the Enrichment of Lymphocytes from Apheresis Residues) but do so at the expense of cell viability and yield (Chiche-Lapierre, Cytotherapy 18(6):S47 (2016)). Thus, there is a need for more efficient processes.

### Summary of the Invention

The present invention is directed, *inter alia,* to *in vitro* methods of collecting and rapidly processing cells, particularly cells that have therapeutic uses. The methods rely on Deterministic Lateral Displacement (DLD), a process that involves flowing a sample through a microfluidic device containing a specifically designed array of microposts that are tilted at a small angle from the direction of fluid flow (Davis, et al., Proc. Natl. Acad. Sci. USA 103:14779-14784 (2006); Inglis, et al., Lab Chip 6:655-658 (2006); Chen, et al., Biomicrofluidics. 9(5):054105 (2015)). Cells larger than the target size of the micropost array may be gently deflected ("bumped") by the microposts into a stream of clean buffer, effectively separating them from smaller, nondeflected cells and particles, while simultaneously washing the cells in a process that is non-injurious. Advantageous characteristics of DLD with respect to cell processing are described in Table 1:

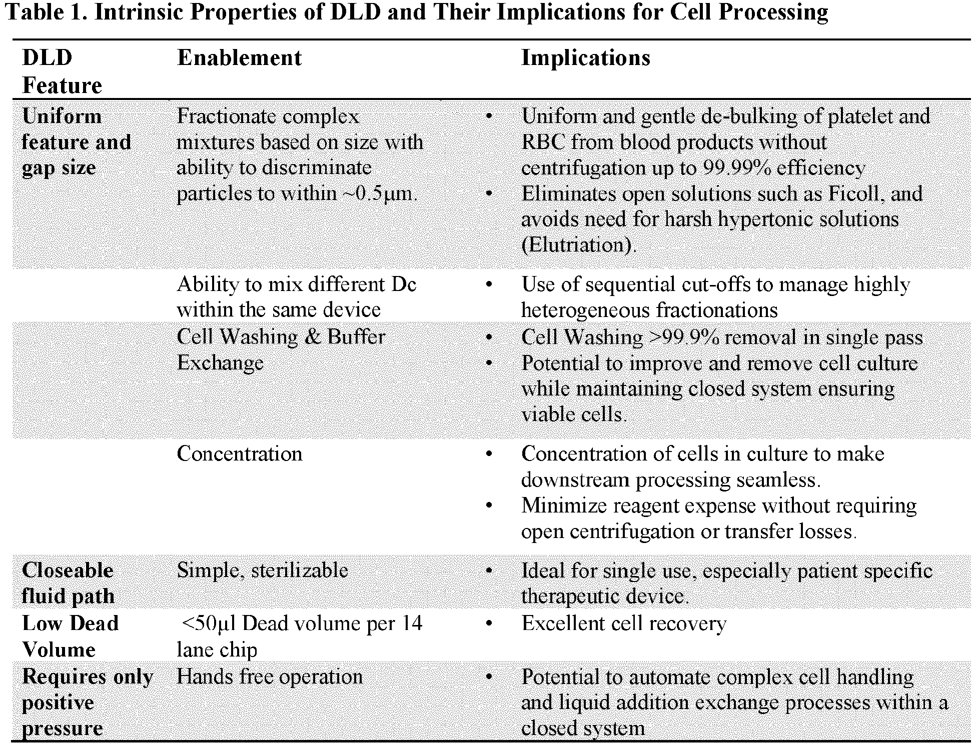

### Methods for Making CAR T Cells

The invention includes a method of producing CAR T cells by obtaining a crude fluid composition comprising T cells (especially natural killer T cells and memory T cells) and performing DLD on the composition using a microfluidic device. Generally, the crude fluid composition comprising T cells will be an apheresis or leukapheresis product derived from the blood of a patient and containing leukocytes.

The microfluidic device must have at least one channel extending from a sample inlet to one or more fluid outlets, wherein the channel is bounded by a first wall and a second wall opposite from the first wall. An array of obstacles is arranged in rows in the channel, each subsequent row of obstacles being shifted laterally with respect to a previous row. These obstacles are disposed in a manner such that, when the crude fluid composition comprising T cells is applied to an inlet of the device and fluidically passed through the channel, the T cells flow to one or more collection outlets where an enriched product is collected and other cells (e.g., red blood cells, and platelets) or other particles of a different (generally smaller) size than the T cells flow to one or more waste outlets that are separate from the collection outlets. Once obtained, the T cells are genetically engineered to produce chimeric antigen receptors (CARs) on their surface using procedures well established in the art. These receptors should generally bind antigens that are on the surface of a cell associated with a disease or abnormal condition. For example, the receptors may bind antigens that are unique to, or overexpressed on, the surface of cancer cells. In this regard, CD19 may sometimes be such an antigen.

The genetic engineering of CAR-expressing T cells will generally comprise transfecting or transducing T cells with nucleic acids and, once produced, the CAR T cells may be expanded in number by growing the cells *in vitro.* Activators or other factors may be added during this process to promote growth, with IL-2 and IL-15 being among the agents that may be used. The yield of T cells expressing chimeric receptors on their surface after DLD, recombinant engineering and expansion, should, in some embodiments be at least 10% greater than T cells prepared in the same manner but not subjected to DLD and preferably at least 20, 30, 40 or 50% greater. Similarly, in some embodiments, the yield of T cells expressing the chimeric receptors on their surface should be at least 10% greater than T cells isolated by Ficoll centrifugation and not subjected to DLD and preferably at least 20, 30, 40 or 50% greater.

Chimeric receptors will typically have a) an extracellular region with an antigen binding domain; b) a transmembrane region and c) an intracellular region. The cells may also be recombinantly engineered with sequences that provide the cells with a molecular switch that, when triggered, reduce CAR T cell number or activity. In a preferred embodiment, the antigen binding domain is a single chain variable fragment (scFv) from the antigen binding regions of both heavy and light chains of a monoclonal antibody. There is also preferably a hinge region of 2-20 amino acids connecting the extracellular region and the transmembrane region. The transmembrane region may have CD3 zeta, CD4, CD8, or CD28 protein sequences and the intracellular region should have a signaling domain, typically derived from CD3-zeta, CD137 or a CD28. Other signaling sequences may also be included that serve to regulate or stimulate activity.

After obtaining the crude fluid composition comprising T cells, the T cells may, for the reasons discussed above, be bound to one or more carriers in a way that promotes DLD separation. This will preferably take place before performing DLD. However, it may also occur after performing DLD and either before or after cells are transfected or transduced for the first time. In a preferred embodiment, the carriers should comprise on their surface an affinity agent (e.g., an antibody, activator, hapten or aptamer) that binds with specificity to T cells, preferably natural killer T cells. The term "specificity" as used in this context means that the carriers bind preferentially to the desired T cells as compared to any other cells in the composition. For example, the carriers may bind to 100 or 1000 CD8+ T cells for each instance in which it binds a different type of cell.

Carriers may, in some embodiments, have a spherical shape and be made of either biological or synthetic material, including collagen, polysaccharides including polystyrene, acrylamide, alginate and magnetic material. In addition, carriers may act in a way that complements DLD separation.

In order to aid in achieving a separation, the diameter of the complex formed between T cells and carriers should preferably be at least 20% larger than the uncomplexed T cells and preferably at least 50% larger, at least twice as large or at least ten times as large. This increase in size may be either due to the binding of a single large carrier to the cells or due to the binding of several smaller carriers. Binding may involve using: a) only carriers with a diameter at least as large (or in other embodiments, at least twice as large or at least ten times as large) as that of the T cells; b) only carriers with a diameter no more than 50% (or in other embodiments, no more than 25% or 15%) as large as that of the T cells; or c) mixtures of large and small carriers with these size characteristics (*e.g.*, there may be one group of carriers with a diameter at least as large (or at least twice or ten times as large) as the T cells and a second group of carriers with a diameter no more than 50% (or no more than 25% or 15%) as large as that of the T cells. Typically a carrier will have a diameter of 1-1000 µm (and often in the range of 5-600 or 5-400 µm). Ideally, the complexes will be separated from uncomplexed cells or contaminants by DLD on a microfluidic device having an array of obstacles with a critical size lower than the size of the complexes but higher than the size of uncomplexed non-target cells or contaminants.

As discussed above in connection with target cells, the purification of T cells may involve a two step process. For example, DLD may be performed on T cells that are not bound to carriers using an array of obstacles with a critical size smaller than the T cells. A composition containing the separated T cells together with other cells or particles may then be recovered and bound to one or more carriers in a way that promotes DLD separation and in which T cells are bound with specificity. The complexes thereby formed may then be separated on an array of obstacles with a critical size smaller than the complexes but larger than uncomplexed cells. In principle, the DLD steps could be performed in either order, *i.e.,* it might be performed on the complexes first or on the uncomplexed T cells first.

Preferably, no more than four hours (and, more preferably, no more than three, two or one hour(s)) should elapse from the time that the obtaining of the crude fluid composition comprising T cells is completed (e.g., from the time that apheresis or leukapheresis is completed) until the T cells are bound to a carrier. In addition, no more than five hours (and preferably no more than four hours, three or two hours) should elapse from the time that the obtaining of T cells is completed until the first time that T cells are transfected or transduced. Ideally, all steps in producing the CAR T cells are performed at the same facility where the crude fluid composition comprising T cells is obtained and all steps are completed in no more than four (and preferably no more than three) hours and without the cells being frozen.

### Collection and Processing of Cells

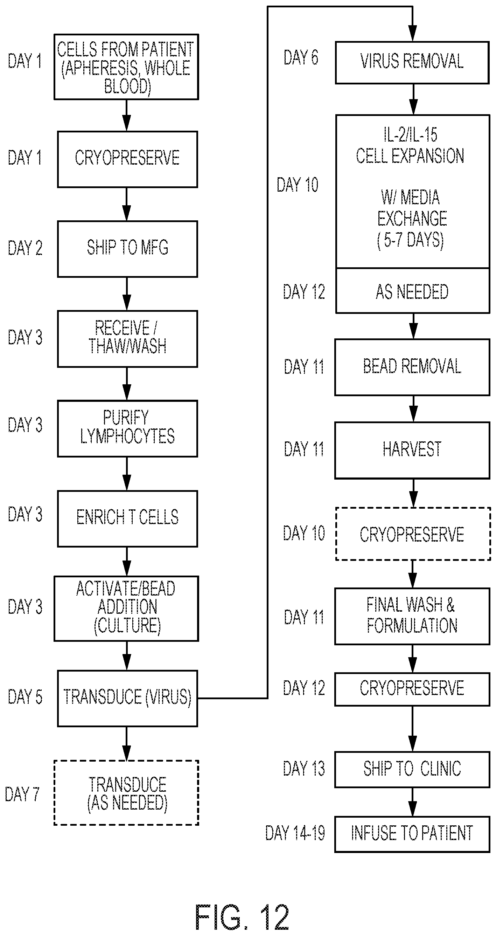

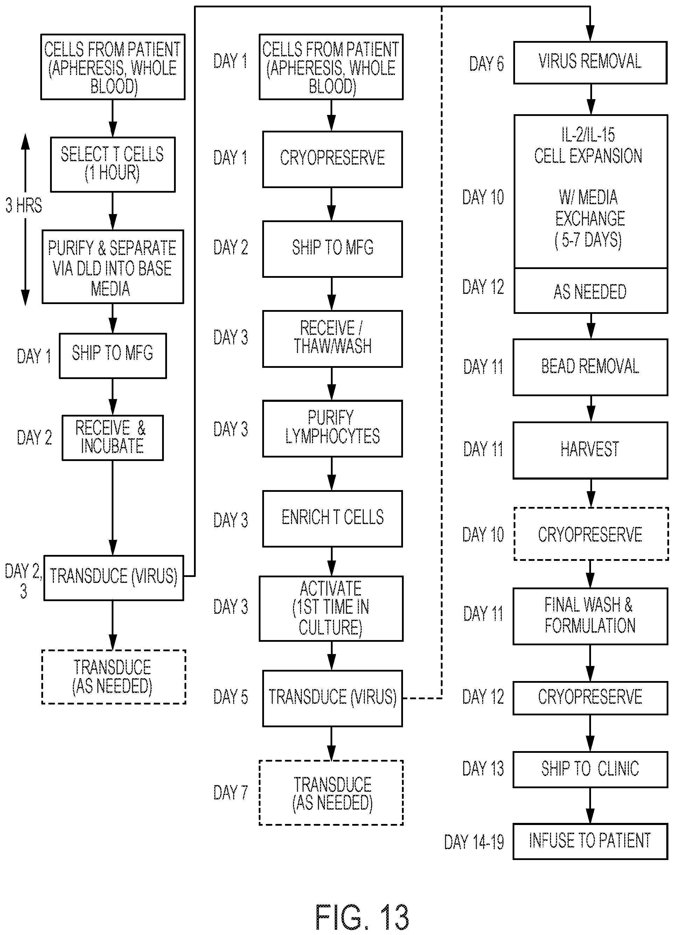

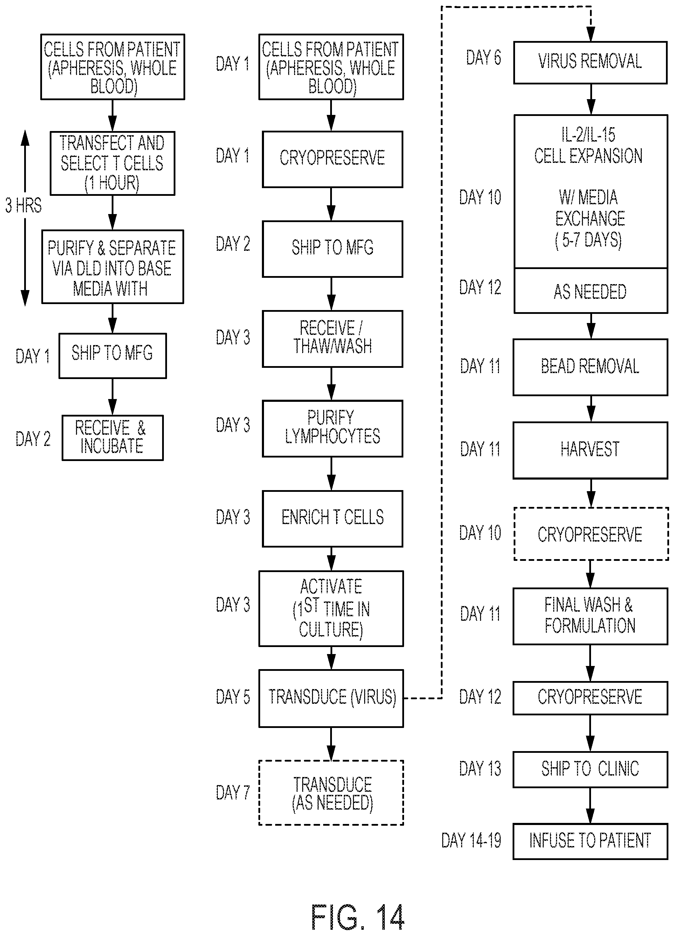

Also described are protocols for collecting and processing cells from a patient which are designed to process cells quickly, and which can generally be performed at sites where the cells are collected. The protocols may be used as a part of the methods for preparing target cells and CAR T cells described above. Aspects of some of these protocols are illustrated in figures 13 and 14 and may be contrasted with the protocol shown in figure 12. In the particular procedures illustrated, a composition obtained by apheresis of whole blood is obtained and T cells in the composition are then selected. The term "selected" in this context means that the T cells are bound by agents that recognize the T cells with specificity (as defined above). DLD is then used to isolate the selected T cells and transfer these cells into a chosen fluid medium.

Also described is a method of collecting target cells by: a) obtaining a crude fluid composition comprising the target cells from a patient; and b) performing Deterministic Lateral Displacement (DLD) on the crude fluid composition to obtain a composition enriched in target cells wherein either before, or after DLD, the target cells are bound to a carrier in a way that promotes DLD separation. For example, a carrier may be used that has on its surface an affinity agent (e.g., an antibody, activator, hapten or aptamer) that binds with specificity (as defined above) to the target cells.

Carrier may, if desired, be bound to target cells during the time that the cells are being collected from the patient and no more than five hours (and preferably no more than four, three, two or one hour(s)) should elapse from the time that the obtaining of the crude fluid composition comprising target cells is completed until the target cells are bound to the carrier.

The diameter of the complex formed between target cells and one or more carriers should preferably be at least 20% larger than the uncomplexed cells and preferably at least 50% larger, at least twice as large or at least ten times as large. This increase in size may be either due to the binding of a single large carrier to the target cells or due to the binding of several smaller carriers. Binding may involve using: i) only carriers with a diameter at least as large (or in other embodiments, at least twice as large or at least ten times as large) as that of the target cells; ii) only carriers with a diameter no more than 50% (or in other embodiments, no more than 25% or 15%) as large as that of the target cells; or iii) mixtures of large and small carriers with these size characteristics (*e.g*., there may be one group of carriers with a diameter at least as large (or at least twice or ten times as large) as the target cells and a second group of carriers with a diameter no more than 50% (or no more than 25% or 15%) as large as that of the target cells. Typically a carrier will have a diameter of 1-1000 µm (and often in the range of 5-600 or 5-400 µm). Ideally the complexes would be separated from other cells or contaminants by DLD on a microfluidic device having an array of obstacles with a critical size lower than the size of the complexes but higher than the size of uncomplexed cells or contaminants.

The crude fluid composition comprising target cells may be obtained by performing apheresis or leukapheresis on blood from the patient. This composition may include one or more additives that act as anticoagulants or that prevent the activation of platelets. Examples of such additives include ticlopidine, inosine, protocatechuic acid, acetylsalicylic acid, and tirofiban alone or in combination.

The microfluidic devices must have at least one channel extending from a sample inlet to one or more fluid outlets, wherein the channel is bounded by a first wall and a second wall opposite from the first wall. There must also be an array of obstacles arranged in rows in the channel, with each subsequent row of obstacles being shifted laterally with respect to a previous row such that, when said crude fluid composition comprising target cells is applied to an inlet of the device and fluidically passed through the channel, target cells flow to one or more collection outlets where an enriched product is collected and contaminant cells, or particles that are in the crude fluid composition and that are of a different size than the target cells flow to one or more waste outlets that are separate from the collection outlets.

In a particularly preferred embodiment, target cells are T cells selected from the group consisting of: Natural Killer T cells; Central Memory T cells; Helper T cells and Regulatory T cells, with Natural Killer T cells being the most preferred. In alternative preferred embodiments, the target cells are stem cells, B cells, macrophages, monocytes, dendritic cells, or progenitor cells.

In addition to steps a) and b), the method of the invention may include: c) genetically engineering cells by transducing them using a viral vector. Alternatively, the cells may be transfected electrically, chemically or by means of nanoparticles and/or expanded cells in number.. In addition, the collected cells may be cultured and/or cryopreserved. In cases where the target cells are T cells, culturing should generally be carried out in the presence of an activator, preferably an activator that is bound to a carrier. Among the factors that may be included in T cell cultures are IL-2 and IL-15.

In addition to the methods discussed above, the invention includes the target cells produced by the methods.

### Methods of Using DLD for Large Volumes of Leukapheresis Material

One advantage of DLD is that it can be used to process small quantities of material with little increase in volume as well as relatively large quantities of material. The procedure may be used on leukapheresis products that have a small volume due to the concentration of leukocytes by centrifugation as well as in processing a large volume of material.

Thus, in another aspect, the invention is directed to a system for purifying cells from large volume leukapheresis processes in which at least one microfluidic device is used that separates materials by DLD. The objective is to obtain leukocytes that may be used therapeutically or that secrete agents that may be used therapeutically. Of particular importance, the invention includes binding specific types of leukocytes to one or more carriers in a way that promotes and, optionally, also complements DLD separation and then performing DLD on the complex. In this way, specific types of leukocytes may be separated from cells that are about the same size and that, in the absence of complex formation, could not be resolved by DLD. In this regard, a two step procedure as discussed above may sometimes be advantageous in which a one DLD procedure separates unbound leukocytes from smaller material and a another DLD procedure separates a carrier-leukocyte complex from uncomplexed cells. Essentially the same technique can be used in other contexts as well, e.g., on cultured cells, provided that cell specific carriers are available. In all instances, the cells may be recombinantly genetically engineered to alter the expression of one or more of their genes.

For leukapheresis material, the microfluidic devices must have at least one channel extending from a sample inlet to both a "collection outlet" for recovering white blood cells (WBCs) or specific leukocyte-carrier complexes and a "waste outlet" through which material of a different size (generally smaller) than WBCs or uncomplexed leukocytes flow. The channel is bounded by a first wall and a second wall opposite from the first wall and includes an array of obstacles arranged in rows, with each successive row being shifted laterally with respect to a previous row. The obstacles are disposed in a manner such that, when leukapheresis material is applied to an inlet of the device and fluidically passed through the channel, cells or cell complexes are deflected to the collection outlet (or outlets) where an enriched product is collected and material of a different (generally smaller) size flows to one or more separate waste outlets.

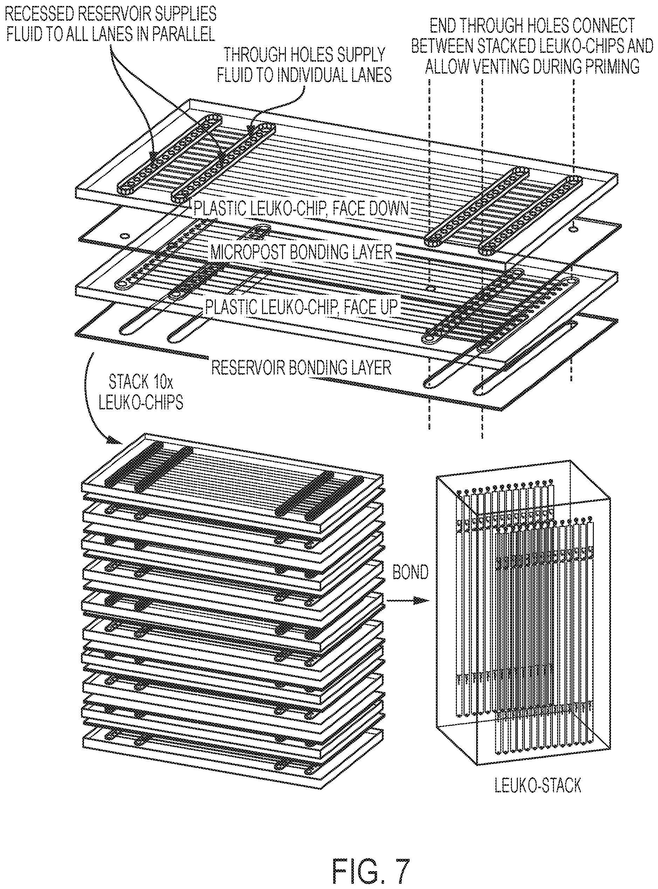

In order to facilitate the rapid processing of large volumes of starting material, the obstacles in microfluidic devices may be designed in the shape of diamonds or triangles and each device may have 6-40 channels. In addition, the microfluidic devices may be part of a system comprising 2-20 microfluidic devices (see Fig. 7). Individual devices may be operated at flow rates of 14 ml/hr but flow rates of at least 25 ml/hr (preferably at least 40, 60, 80 or 100 ml per hour) are preferable and allow large sample volumes (at least 200 ml and preferably 400-600 ml) to be processed within an hour.

### Separation of Adherent Cells

Also described is a method of obtaining adherent target cells, preferably cells of therapeutic value, e.g., adherent stem cells, by: a) obtaining a crude fluid composition comprising the adherent target cells from a patient; and b) performing Deterministic Lateral Displacement (DLD) to obtain a composition enriched in the adherent target cells. During this process, the adherent target cells may be bound to one or more carriers in a way that promotes or complements DLD separation. For example carriers may have on their surface an affinity agent (*e.g*., an antibody, activator, hapten or aptamer) that binds with specificity (as defined above) to the adherent target cells and may be transfected or transduced with nucleic acids designed to impart on the cells a desired phenotype, e.g., to express a chimeric molecule (preferably a protein that makes the cells of greater therapeutic value).

Carriers may be added at the time that the crude fluid composition is being collected or, alternatively after collection is completed but before DLD is performed for the first time. In a second alternative, DLD may be performed for a first time before carrier is added. For example, if the adherent cell has a size less than the critical size, the crude fluid composition may be applied to the device before the carrier is added, the adherent cell may be recovered, the cells may then be attached to one or more carriers to form a complex that is larger than the critical size of a device, a second DLD step may then be preformed and the carrier adherent cell complexes may be collected.

Preferably, no more than three hours (and more preferably no more than two hours, or one hour) elapse from the time that the obtaining of the crude fluid composition from the patient is completed until the adherent cell is bound to a carrier for the first time. In another preferred embodiment, no more than four hours (and preferably no more than three or two hours) elapse from the time that the obtaining of the crude fluid composition from the patient is complete until the first time that the adherent cell or a carrier adherent cell complex is collected from the device for the first time.

The methodology described above may be used to separate adherent target cells, *e.g*., adherent stem cells, from a plurality of other cells. The method involves: a) contacting a crude fluid composition comprising the adherent target cells and the plurality of other cells, wherein the adherent target cells are at least partially associated with one or more carriers in a way that promotes DLD separation and form carrier associated adherent target cell complexes, wherein the complexes comprise an increased size relative to the plurality of other cells, and wherein the size of the carrier associated adherent cell complexes is preferably at least 50% greater than a critical size, and other, uncomplexed cells comprise a size less than the critical size; b) applying the crude fluid composition containing the carrier associated adherent cell complexes to a device, wherein the device comprises an array of obstacles arranged in rows, wherein the rows are shifted laterally with respect to one another, wherein the rows are configured to deflect cells or complexes greater than or equal to the critical size in a first direction and cells or complexes less than the critical size in a second direction; c) flowing the crude fluid composition comprising the carrier associated adherent target cell complexes through the device, wherein the complexes are deflected by the obstacles in the first direction, and uncomplexed cells are deflected in the second direction, thereby separating the carrier associated adherent cell complexes from the other uncomplexed cells; d) collecting a fluid composition comprising the separated carrier associated adherent target cell complexes.

The diameter of the complex formed between adherent target cells and one or more carriers should preferably be at least 20% larger than the uncomplexed cells and preferably at least 50% larger, at least twice as large or at least ten times as large. This increase in size may be either due to the binding of a single large carrier to the adherent target cells or due to the binding of several smaller carriers. Binding may involve using: a) only carriers with a diameter at least as large (or in other embodiments, at least twice as large or at least ten times as large) as that of the adherent target cells; b) only carriers with a diameter no more than 50% (or in other embodiments, no more than 25% or 15%) as large as that of the adherent target cells; or c) mixtures of large and small carriers with these size characteristics (*e.g*., there may be one group of carriers with a diameter at least as large (or at least twice or ten times as large) as the adherent target cells and a second group of carriers with a diameter no more than 50% (or no more than 25% or 15%) as large as that of the adherent target cells. Typically a carrier will have a diameter of 1-1000 µm (and often in the range of 5-600 or 5-400 µm).

The carriers may be made of any of the materials that are known in the art for the culturing of adherent cells including polypropylene, polystyrene, glass, gelatin, collagen, polysaccharides, plastic, acrylamide and alginate. They may be uncoated or coated with materials that promote adhesion and growth (e.g., serum, collagen, proteins or polymers) and may have agents (*e.g*., antibodies, antibody fragments, substrates, activators or other materials) attached to their surfaces. In some embodiments, the diluent can be growth media, the steps can be performed sequentially and, after step (d), buffer exchange can be performed.

Examples of specific adherent cells that may be isolated in the methods described above include: an MRC-5 cell; a HeLa cell; a Vero cell; an NIH 3T3 cell; an L929 cell; a Sf21 cell; a Sf9 cell; an A549 cell; an A9 cell; an AtT-20 cell; a BALB/3T3 cell; a BHK-21 cell; a BHL-100 cell; a BT cell; a Caco-2 cell; a Chang cell; a Clone 9 cell; a Clone M-3 cell; a COS-1 cell; a COS-3 cell; a COS-7 cell; a CRFK cell; a CV-1 cell; a D-17 cell; a Daudi cell; a GH1 cell; a GH3 cell; an HaK cell; an HCT-15 cell; an HL-60 cell; an HT-1080 cell; a HEK cell, HT-29 cell; an HUVEC cell; an I-10 cell; an IM-9 cell; a JEG-2 cell; a Jensen cell; a Jurkat cell; a K-562 cell; a KB cell; a KG-1 cell; an L2 cell; an LLC-WRC 256 cell; a McCoy cell; a MCF7 cell; a WI-38 cell; a WISH cell; an XC cell; a Y-1 cell; a CHO cell; a Raw 264.7 cell; a HEP G2 cell; a BAE-1 cell; an SH-SY5Y cell, and any derivative thereof.

### Separation of Cells Bound to an Activator

The invention also includes methods of purifying cells capable of activation using the procedures described above. In a preferred embodiment, the invention is directed to a method of separating an activated cell from a plurality of other cells by: a) contacting a crude fluid composition comprising a cell capable of activation and the plurality of other cells with one or more carriers, in a way that promotes DLD separation, wherein one or more of the carriers comprise a cell activator, wherein one or more carriers are at least partially associated with the cell capable of activation by the cell activator upon or after contact to generate a carrier associated cell complex, wherein the association of the cell activator with the cell capable of activation at least partially activates the cell capable of activation, wherein the carrier associated cell complex comprises an increased size relative to other cells, and wherein a size of the carrier associated cell complex is greater than or equal to a critical size, and the cells in the plurality of other cells comprise a size less than the critical size; b) applying the crude fluid composition to a device, wherein the device comprises an array of obstacles arranged in rows; wherein the rows are shifted laterally with respect to one another, wherein the rows are configured to deflect a particle greater than or equal to the critical size in a first direction and a particle less than the critical size in a second direction; c) flowing the sample through the device, wherein the carrier associated cell complex is deflected by the obstacles in the first direction, and the cells in the plurality of other cells are deflected in the second direction, thereby separating the activated cell from the plurality of other cells. The fluid composition comprising the separated carrier associated cell complex may then be collected. During this process the cells may optionally be transfected or transduced with nucleic acids designed to impart on the cells a desired phenotype, *e.g*., to express a chimeric molecule (preferably a protein that makes the cells of greater therapeutic value).

The cell capable of activation may be selected from the group consisting of: a T cell, a B cell, a macrophage, a dendritic cell, a granulocyte, an innate lymphoid cell, a megakaryocyte, a natural killer cell, a thrombocyte, a synoviocyte, a beta cell, a liver cell, a pancreatic cell; a DE3 lysogenized cell, a yeast cell, a plant cell, and a stem cell.

The cell activator may be selected from the group consisting of: an antibody or antibody fragment, CD3, CD28, an antigen, a helper T cell, a receptor, a cytokine, a glycoprotein, and any combination thereof. In other embodiments, the activator may be a small compound and may be selected from the group consisting of insulin, IPTG, lactose, allolactose, a lipid, a glycoside, a terpene, a steroid, an alkaloid, and any combination thereof.

In a preferred embodiment, the cell capable of activation has been collected from a patient as part of a crude fluid composition comprising the cell capable of activation and a plurality of other cells, wherein no more than four hours (and preferably no more than three hours, two hours or one hour) elapse from the time that the obtaining of the crude fluid composition from the patient is completed until the cell capable of activation is bound to the carrier. It is also preferable that no more than four hours elapse from the time that the obtaining of the crude fluid composition from the patient is completed until step c) is completed. Alternatively, the method may be altered by binding activator before collection of cells begins.

Preferably, the diameter of the complex formed between a cell capable of activation and one or more carriers should be at least 20% larger than the uncomplexed cells and more preferably at least 50% larger, at least twice as large or at least ten times as large. This increase in size may be either due to the binding of a single large carrier to the cell capable of activation or due to the binding of several smaller carriers. Binding may involve using: a) only carriers with a diameter at least as large (or in other embodiments, at least twice as large or at least ten times as large) as that of the cell capable of activation; b) only carriers with a diameter no more than 50% (or in other embodiments, no more than 25% or 15%) as large as that of the cell capable of activation; or c) mixtures of large and small carriers with these size characteristics (*e.g*., there may be one group of carriers with a diameter at least as large (or at least twice or ten times as large) as the cell capable of activation and a second group of carriers with a diameter no more than 50% (or no more than 25% or 15%) as large as that of the cell capable of activation. Typically a carrier will have a diameter of 1-1000 µm (and often in the range of 5-600 or 5-400 µm).

### Brief Description of the Drawings

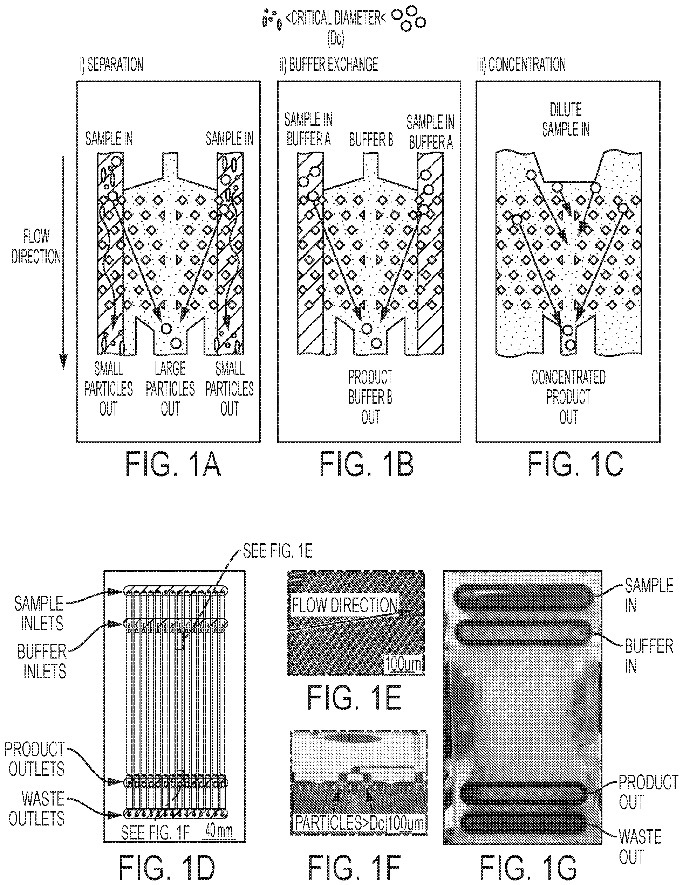

**Figures 1A-1G:** Figures 1A-1C illustrate different operating modes of DLD. This includes: i) Separation (Fig. 1A), ii) Buffer Exchange (Fig. 1B) and iii) Concentration (Fig. 1C). In each mode, essentially all particles above a critical diameter are deflected in the direction of the array from the point of entry, resulting in size selection, buffer exchange or concentration as a function of the geometry of the device. In all cases, particles below the critical diameter pass directly through the device under laminar flow conditions and subsequently off the device. Figure 1D shows a 14 lane DLD design used in separation mode. The full length of the depicted array and microchannel is 75 mm and the width is 40 mm, each individual lane is 1.8 mm across. Figures 1E-1F are enlarged views of the plastic diamond post array and consolidating collection ports for the exits. Figure 1G depicts a photo of a leukapheresis product being processed using a prototype device at 10 PSI, i.e. 68.95 kilopascal.

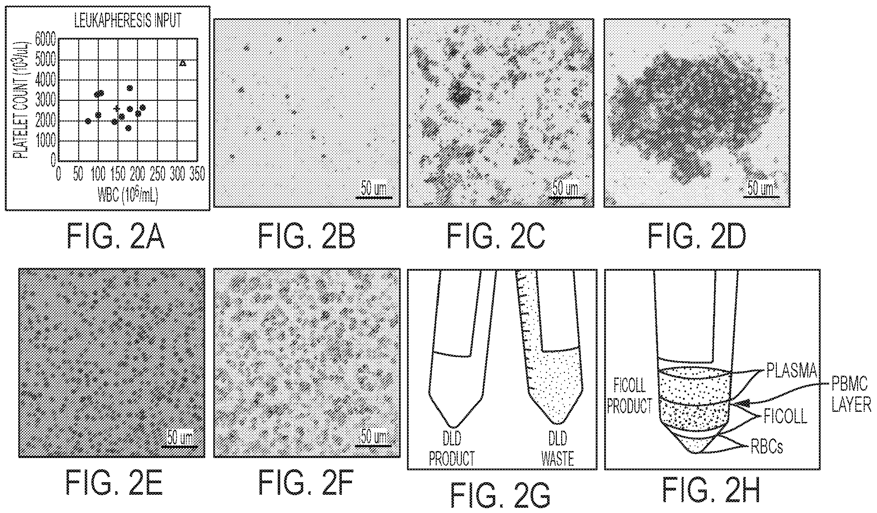

**Figures 2A-2H:** Figure 2A is a scatter plot showing the range of normal donor platelet and WBC cell counts used in this study. Mean counts of WBC: 162.4 × 10⁶/mL and Platelets: 2718 × 10³/µL respectively (+). The outlier sample (▲), clogged the 20µm prefilter and was excluded from the data set. Input sample shown (Figs. 2C and 2D). Representative 24-hour old normal donor leukapheresis input (Fig. 2B) and PBMC product processed by either a 14-lane diamond post DLD at 10 PSI, i.e. 68.95 kilopascal (Fig. 2E) or Ficoll-Hypaque (Fig. 2F). Representative DLD product (Fig. 2G) and Ficoll (Fig. 2H) from the same Leukapheresis donor (#37). Input (Figs. 2B, 2C, 2D) and product fractions (Fig. 2E and 2F) were fixed and stained on slides with CD41-FITC (platelets) plus CD45-Alexa647 (WBC) and counter-stained with DAPI (nuclear DNA).

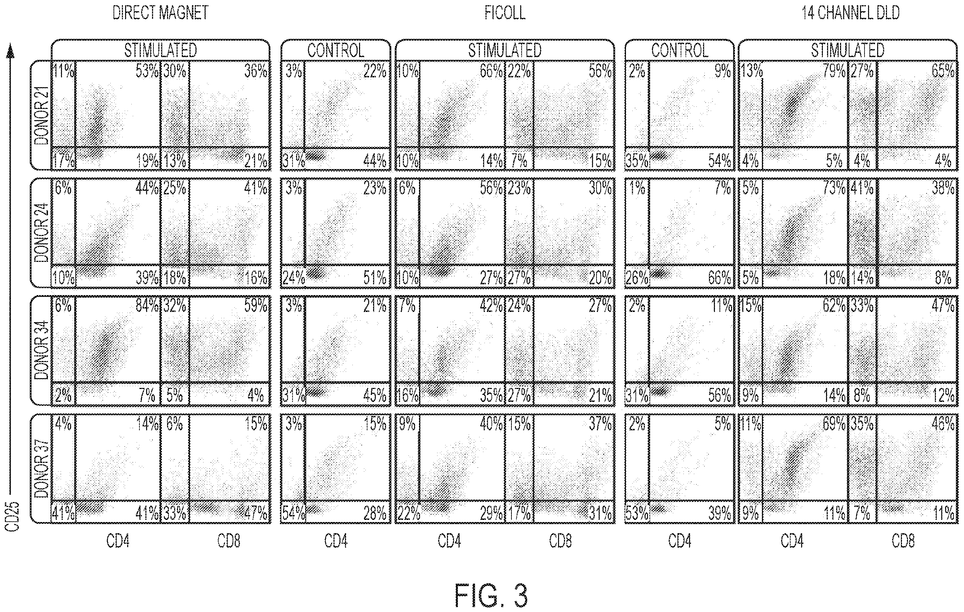

**Figure 3****:** This figure concerns the consistency of cell activation in DLD vs. Ficoll and Direct Magnet approaches (CD4, CD8 vs CD25 Day 8). Cell activation and Phenotypic profile shows a shift during expansion towards classic central memory T cell associated phenotype (Day 8). Cells were counted and de-beaded as described previously. At each time point ~100,000 cells were stained with CD3-BV421, CD45RA-BV605, CD95-FITC, CD279-PE, CD25-APC, CD4-Alexa 700, and CD8-APC-Cy7, incubated for 30 at room temperature in the dark and washed with 10 volumes of PBS prior to centrifugation and fixation in 1.0% Para-formaldehyde in PBS. Samples were acquired on a BD FACSAria, and analyzed using a CD3 and forward and side scatter gate using FlowLogic software.

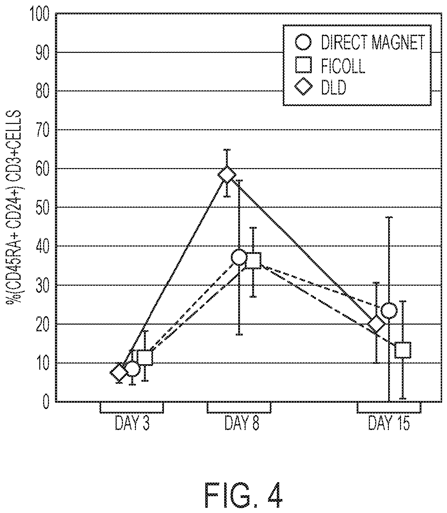

**Figure 4:** Figure 4 is a graph depicting rapid gain of memory cell phenotype and consistent activation of samples via DLD compared to Ficoll & Direct Magnet. Plot of % CD45RA-, CD25+ cells measuring conversion to T cell activation and conversion via CD45 RO status is shown. Cells were fed 200 Units IL-2/mL culture at Day 3 and again at day 8 only as the experiment was designed to address initial ability to expand.

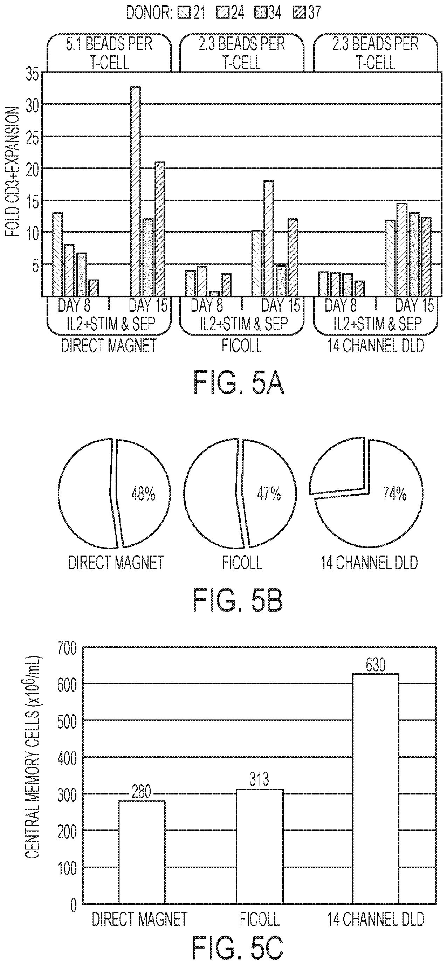

**Figures 5A-5C****:** These figures concern the Fold Expansion of CD3 cells (× 10⁶) from DLD, Ficoll and Direct Magnet. Aliquots of DLD product and Ficoll cells were incubated with CD3/CD28 beads following Thermo-Fisher CTS protocol using a T cell density of 1 × 10⁷ T cells/mL. A ratio of ~2.5 Beads/T cell and for the Direct Magnet using ~5.0 Beads/T cell was used, cells and beads were incubated on a rotary mixer for 60 min prior to magnetic separation. Either stimulated or unstimulated (unseparated PMBC) cells were diluted in complete media (RPMI-1640 + 10% FBS + antibiotics without IL-2) to 0.5 × 10⁶/ mL and were plated in time point specific reactions to avoid any disturbance of the cultures at intermediate time points. On Day 3, 200 ill of IL-2/mL was added to the stimulated and separated arm per manufacturer's recommendation. Cell counts were determined on Day 3, 8, 15 after de-beading using manufacturers protocol (pipetting) by Coulter count (Scepter) and verified by bead based absolute counting using flow cytometry on a BD FACSCalibur using a no-wash approach with a fluorescence threshold on CD45 and staining with CD3- FITC, CD45-PerCP and using the DNA stain DRAQ5 to ensure effective discrimination of doublets and any cells with beads still attached. Correlation between counting methods was acceptable with a slope of 0.95, R2=0.944. Media was added to the cultures to maintain cell densities in an acceptable range (<3.0 × 10⁶/mL) on Days 6, and 9. Day 15 data point for the donor 21 was lost due to contamination. Averages or % CV's shown in horizontal bars as indicated (Fig. 5A). Fig. 5B shows the percentage of T central memory cells (day 15) and Fig. 5C shows the number of T central memory cells (day 15).

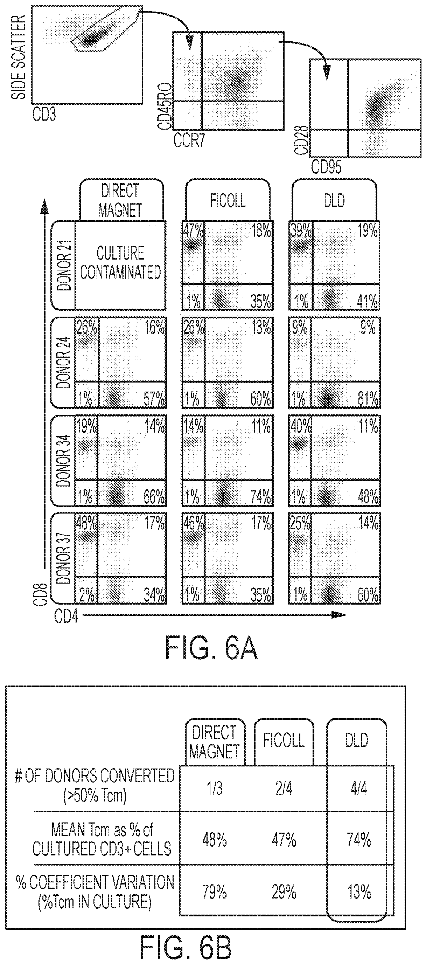

**Figure 6A-6B:** Figures 6A-6B concern cytometric analysis of T central memory cells and the number of central memory cells produced. Fig. 6A: T Central Memory Cells: CD3+ T cells were gated on a singlet gate followed by a CD3 v Side scatter and central memory phenotyping using 4 parameter gate of CD45RO, CCR7, CD28 and CD95 to define the central memory population. The population was back gated to display central memory cells, in red, as fraction of T cells. Non-red cells represented all non central memory T cells. Fig. 6B: Phenotype Conversion and Key Metrics (Day 15): Key metrics show # of donors where the number of central memory cells is >50%, with the average and % CV's associated with the central memory expansion.
**Figure 7:** Figure 7 is a schematic showing how current individual chips have been designed to be stackable in layers to achieve throughput as demanded by any particular application using established manufacturing approaches. Injection molded layers are planned as systems are developed.

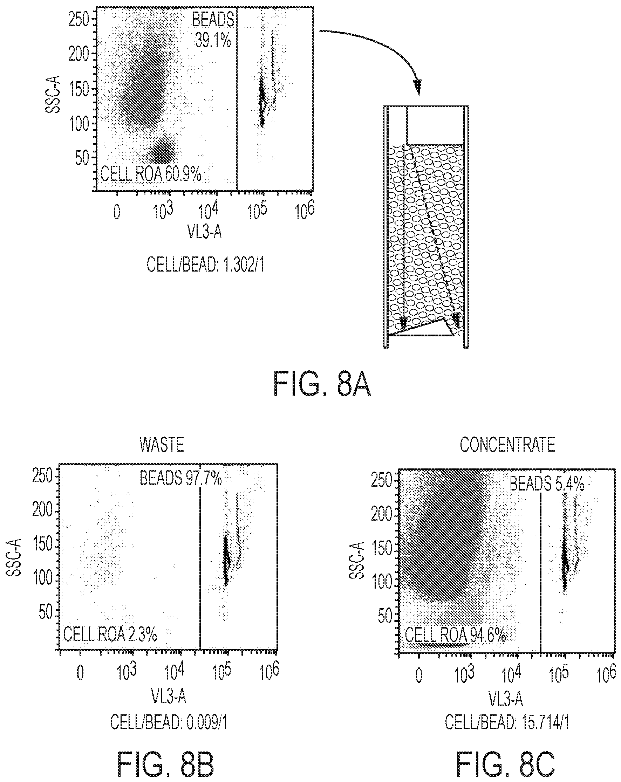

**Figures 8A-8C****:** These are supplemental figures showing the concentration of WBC via DLD. Fig. 8A: DLD Product Derived from Whole Blood: Whole blood was passed over first DLD to remove erythrocytes. A second, in line, concentrating DLD, designed to achieve a concentration factor of 12, was connected to the product output of the separating DLD. Equal volumes of product and waste were added to tubes with equal numbers of absolute count beads and analyzed by flow cytometry. The resulting relative cell:bead ratio for Waste (Fig. 8B) and for Concentrate (Fig. 8C) was calculated compared to the input material to determine fold concentration. Leukocytes were stained with CD45 PerCP and 1mM DRAQ5, which was used as a fluorescence threshold to acquire both the beads and the leukocytes. 5000 bead events acquired. (all reagents eBioscience). Designed Concentration Factor: 12.0x; Observed relative concentration: 15.714/1.302 = 12.07x

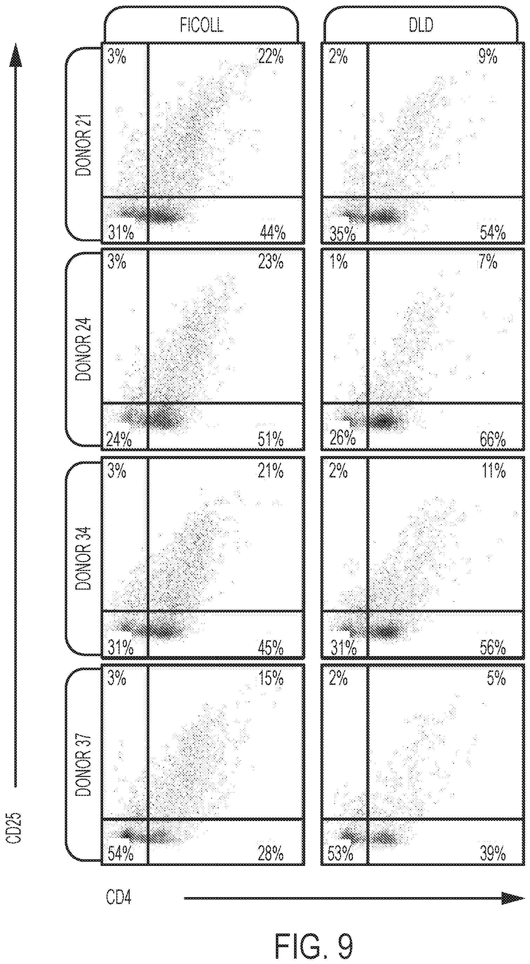

**Figure 9:** Figure 9 is a supplemental figure on the expression of CD25 and CD4 on unstimulated CD3+ T Cells purified by either DLD or Ficoll methods (Day 8). Cells were prepared as described and analyzed as in Figure 3. Mean CD4+25+: Ficoll: 20.25%; DLD: 8%.

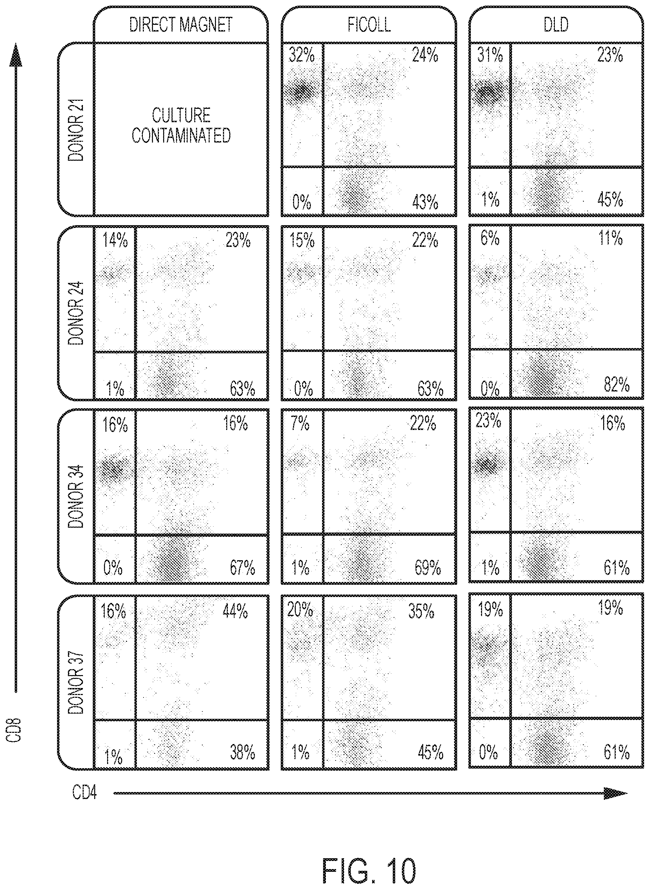

**Figure 10****:** This is a supplemental figure on the allocation of IL-2 expanded central memory T cells by major subsets. In the original figures: CD8 (Green), CD4 (Blue), CD4+CD8+ (Red) Central memory cells were sequentially gated: CD3+, CD45RO+CCR7+, CD28+CD95+. Relative abundance of CD4 subset driven by IL2 is evident.

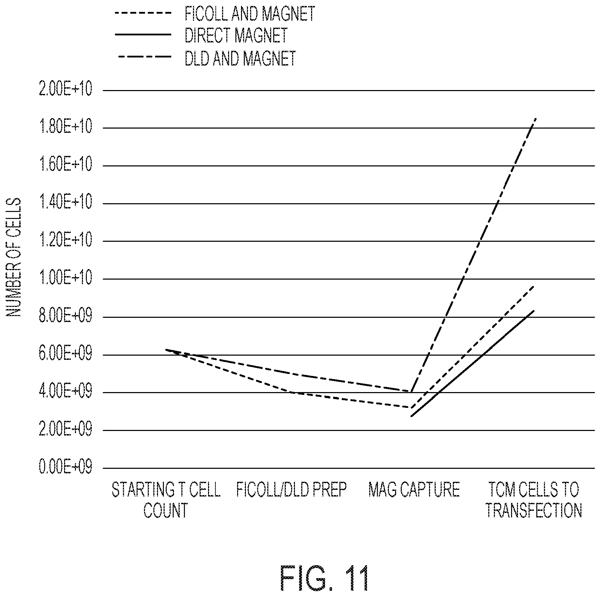

**Figure 11:** Figure 11 is a supplemental figure depicting estimates of the number of central memory T cells, post expansion with IL-2, assuming yields in this study and a typical leukapheresis harvest from a donor with 50×10⁶ WBC cells per/mL and containing 50% CD3 lymphocytes in 250 mL.
**Figure 12:** Figure 12 illustrates a protocol that might, in principle, be used for producing CAR T cells and administering the cells to a patient. It has been included to contrast other procedures discussed herein and does not represent work actually performed.
**Figure 13:** Figure 13 illustrates a proposed protocol for producing CAR T cells that differs from the protocol of Figure 12 in the initial steps of the procedure. The steps in the center portion of the figure are included for purposes of comparison. The diagram is intended to illustrate inventive concepts and does not represent work actually performed.
**Figure 14:** Figure 14 illustrates a second proposed protocol for producing CAR T cells that differs from the protocol of Figure 12 in the initial steps of the procedure. The steps in the center portion of the figure are included for purposes of comparison. As with figures 12 and 13, the diagram is intended to illustrate inventive concepts and does not represent work actually performed.

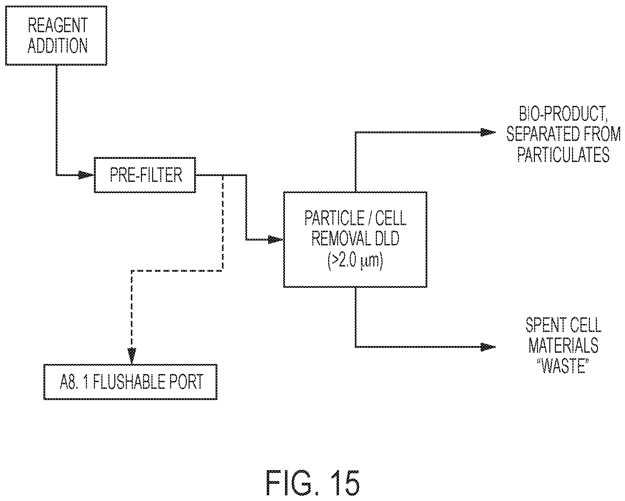

**Figure 15:** Figure 15 shows a schematic of a device for removing secreted products from spent cells.

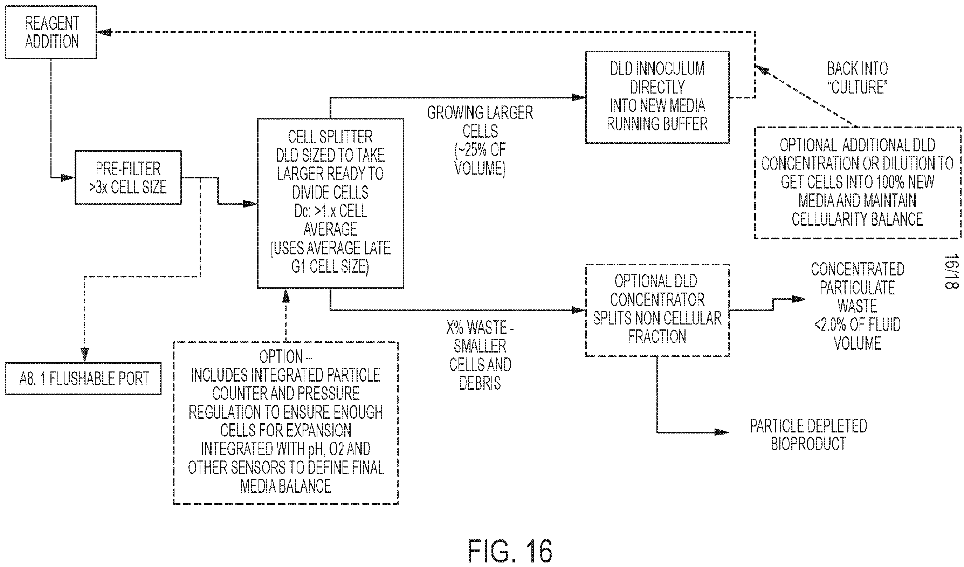

**Figure 16:** Figure 16 shows a schematic of a device for continuous removal of toxic compounds from actively growing cells.

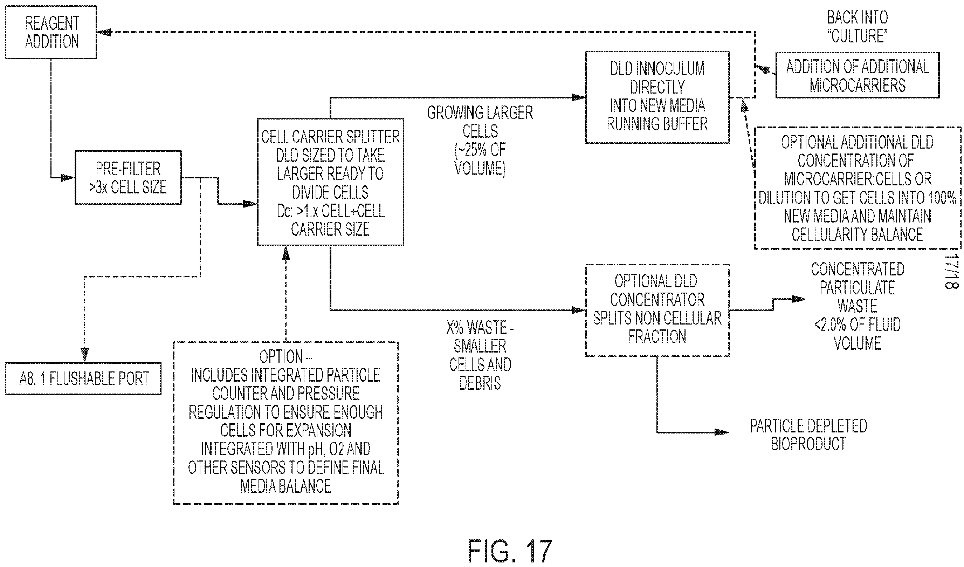

**Figure 17:** Figure 17 shows a schematic of a device for continuous removal of toxic compounds from actively growing cells with the option of adding carriers between each iteration.

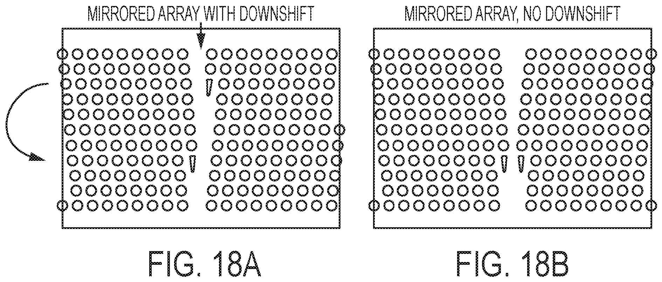

Figure 18A and 18B: Figure 18 A shows an example of a mirrored array of obstacles with a downshift. A central channel is between an array of obstacles on the left and on the right. The central channel can be a collection channel for particles of at least a critical size *(i.e.,* particles of at least a critical size can be deflected by the arrays to the central channels, whereas particles of less than the critical size can pass through the channel with the bulk flow). By downshifting rows, changes in the width of the channel relative to a mirrored array with a downshift can be achieved. The amount of downshift can vary based on the size and/or cross-sectional shape of the obstacles. FIG. 18B illustrates a mirrored array of obstacles with no downshift. An array on the left and an array on the right can deflect particles of at least a critical size to the central channel.

### Definitions

Apheresis: As used herein this term refers to a procedure in which blood from a patient or donor is separated into its components, e.g., plasma, white blood cells and red blood cells. More specific terms are "plateletpheresis" (referring to the separation of platelets) and "leukapheresis" (referring to the separation of leukocytes). In this context, the term "separation" refers to the obtaining of a product that is enriched in a particular component compared to whole blood and does not mean that absolute purity has been attained.

CAR T cells: The term "CAR" is an acronym for "chimeric antigen receptor." A "CAR T cell" is therefore a T cell that has been genetically engineered to express a chimeric receptor.

CAR T cell therapy: This term refers to any procedure in which a disease is treated with CAR T cells. Diseases that may be treated include hematological and solid tumor cancers, autoimmune diseases and infectious diseases.

Carrier: As used herein, the term "carrier" refers an agent, *e.g.,* a bead, or particle, made of either biological or synthetic material that is added to a preparation for the purpose of binding directly or indirectly (*i.e*., through one or more intermediate cells, particles or compounds) to some or all of the compounds or cells present. Carriers may be made from a variety of different materials, including DEAE-dextran, glass, polystyrene plastic, acrylamide, collagen, and alginate and will typically have a size of 1-1000 µm. They may be coated or uncoated and have surfaces that are modified to include affinity agents (*e.g*., antibodies, activators, haptens, aptamers, particles or other compounds) that recognize antigens or other molecules on the surface of cells. The carriers may also be magnetized and this may provide an additional means of purification to complement DLD and they may comprise particles (e.g., Janus or Strawberry-like particles) that confer upon cells or cell complexes non-size related secondary properties. For example the particles may result in chemical, electrochemical, or magnetic properties that can be used in downstream processes, such as magnetic separation, electroporation, gene transfer, and/or specific analytical chemistry processes. Particles may also cause metabolic changes in cells, activate cells or promote cell division.

Carriers that bind "in a way that promotes DLD separation": This term, refers to carriers and methods of binding carriers that affect the way that, depending on context, a cell, protein or particle behaves during DLD. Specifically, "binding in a way that promotes DLD separation" means that: a) the binding must exhibit specificity for a particular target cell type, protein or particle; and b) must result in a complex that provides for an increase in size of the complex relative to the unbound cell, protein or particle. In the case of binding to a target cell, there must be an increase of at least 2 µm (and alternatively at least 20, 50, 100, 200, 500 or 1000% when expressed as a percentage). In cases where therapeutic or other uses require that target cells, proteins or other particles be released from complexes to fulfill their intended use, then the term "in a way that promotes DLD separation" also requires that the complexes permit such release, for example by chemical or enzymatic cleavage, chemical dissolution, digestion, due to competition with other binders, or by physical shearing (*e.g*., using a pipette to create shear stress) and the freed target cells, proteins or other particles must maintain activity; *e.g*., therapeutic cells after release from a complex must still maintain the biological activities that make them therapeutically useful.

Carriers may also bind "in a way that complements DLD separation": This term refers to carriers and methods of binding carriers that change the chemical, electrochemical, or magnetic properties of cells or cell complexes or that change one or more biological activities of cells, regardless of whether they increase size sufficiently to promote DLD separation. Carriers that complement DLD separation also do not necessarily bind with specificity to target cells, *i.e.*, they may have to be combined with some other agent that makes them specific or they may simply be added to a cell preparation and be allowed to bind non-specifically. The terms "in a way that complements DLD separation" and "in a way that promotes DLD separation" are not exclusive of one another. Binding may both complement DLD separation and also promote DLD separation. For example a polysaccharide carrier may have an activator on its surface that increases the rate of cell growth and the binding of one or more of these carriers may also promote DLD separation. Alternatively binding may just promote DLD separation or just complement DLD separation.

Target cells: As used herein "target cells" are the cells that various procedures described herein require or are designed to purify, collect, engineer etc. What the specific cells are will depend on the context in which the term is used. For example, if the objective of a procedure is to isolate a particular kind of stem cell, that cell would be the target cell of the procedure.

Isolate, purify: Unless otherwise indicated, these terms, as used herein, are synonymous and refer to the enrichment of a desired product relative to unwanted material. The terms do not necessarily mean that the product is completely isolated or completely pure. For example, if a starting sample had a target cell that constituted 2% of the cells in a sample, and a procedure was performed that resulted in a composition in which the target cell was 60% of the cells present, the procedure would have succeeded in isolating or purifying the target cell.

Bump Array: The terms "bump array" and "obstacle array" are used synonymously herein and describe an ordered array of obstacles that are disposed in a flow channel through which a cell or particle-bearing fluid can be passed.

Deterministic Lateral Displacement: As used herein, the term "Deterministic Lateral Displacement" or "DLD" refers to a process in which particles are deflected on a path through an array, deterministically, based on their size in relation to some of the array parameters. This process can be used to separate cells, which is generally the context in which it is discussed herein. However, it is important to recognize that DLD can also be used to concentrate cells and for buffer exchange. Processes are generally described herein in terms of continuous flow (DC conditions; *i.e.*, bulk fluid flow in only a single direction). However, DLD can also work under oscillatory flow (AC conditions; *i.e*., bulk fluid flow alternating between two directions).

Critical size: The "critical size" or "predetermined size" of particles passing through an obstacle array describes the size limit of particles that are able to follow the laminar flow of fluid. Particles larger than the critical size can be 'bumped' from the flow path of the fluid while particles having sizes lower than the critical size (or predetermined size) will not necessarily be so displaced. When a profile of fluid flow through a gap is symmetrical about the plane that bisects the gap in the direction of bulk fluid flow, the critical size can be identical for both sides of the gap; however when the profile is asymmetrical, the critical sizes of the two sides of the gap can differ.

Fluid flow: The terms "fluid flow" and "bulk fluid flow" as used herein in connection with DLD refer to the macroscopic movement of fluid in a general direction across an obstacle array. These terms do not take into account the temporary displacements of fluid streams for fluid to move around an obstacle in order for the fluid to continue to move in the general direction.

Tilt angle ε: In a bump array device, the tilt angle is the angle between the direction of bulk fluid flow and the direction defined by alignment of rows of sequential (in the direction of bulk fluid flow) obstacles in the array.

Array Direction: In a bump array device, the "array direction" is a direction defined by the alignment of rows of sequential obstacles in the array. A particle is "bumped" in a bump array if, upon passing through a gap and encountering a downstream obstacle, the particle's overall trajectory follows the array direction of the bump array *(i.e.,* travels at the tilt angle ε relative to bulk fluid flow). A particle is not bumped if its overall trajectory follows the direction of bulk fluid flow under those circumstances.

### Detailed Description of the Invention

The present invention is primarily concerned with the use of DLD in preparing cells that are of therapeutic value. The text below provides guidance regarding methods disclosed herein and information that may aid in the making and use of devices involved in carrying out those methods.

### I. Designing Microfluidic Plates

Cells, particularly cells in compositions prepared by apheresis or leukapheresis, may be isolated by performing DLD using microfluidic devices that contain a channel through which fluid flows from an inlet at one end of the device to outlets at the opposite end. Basic principles of size based microfluidic separations and the design of obstacle arrays for separating cells have been provided elsewhere (see, US 2014/0342375; US 2016/0139012; 7,318,902 and US 7,150,812, which are hereby incorporated herein in their entirety) and are also summarized in the sections below.

During DLD, a fluid sample containing cells is introduced into a device at an inlet and is carried along with fluid flowing through the device to outlets. As cells in the sample traverse the device, they encounter posts or other obstacles that have been positioned in rows and that form gaps or pores through which the cells must pass. Each successive row of obstacles is displaced relative to the preceding row so as to form an array direction that differs from the direction of fluid flow in the flow channel. The "tilt angle" defined by these two directions, together with the width of gaps between obstacles, the shape of obstacles, and the orientation of obstacles forming gaps are primary factors in determining a "critical size" for an array. Cells having a size greater than the critical size travel in the array direction, rather than in the direction of bulk fluid flow and particles having a size less than the critical size travel in the direction of bulk fluid flow. In devices used for leukapheresis-derived compositions, array characteristics may be chosen that result in white blood cells being diverted in the array direction whereas red blood cells and platelets continue in the direction of bulk fluid flow. In order to separate a chosen type of leukocyte from others having a similar size, a carrier may then be used that binds to that cell with in a way that promotes DLD separation and which thereby results in a complex that is larger than uncomplexed leukocytes. It may then be possible to carry out a separation on a device having a critical size smaller than the complexes but bigger than the uncomplexed cells.

The obstacles used in devices may take the shape of columns or be triangular, square, rectangular, diamond shaped, trapezoidal, hexagonal or teardrop shaped. In addition, adjacent obstacles may have a geometry such that the portions of the obstacles defining the gap are either symmetrical or asymmetrical about the axis of the gap that extends in the direction of bulk fluid flow.

### II. Making and Operating Microfluidic Devices

General procedures for making and using microfluidic devices that are capable of separating cells on the basis of size are well known in the art. Such devices include those described in US 5,837,115; US 7,150,812; US 6,685,841; US 7,318,902; 7,472,794; and US 7,735,652; all of which are hereby incorporated by reference in their entirety. Other references that provide guidance that may be helpful in the making and use of devices for the present invention include: US 5,427,663; US 7,276,170; US 6,913,697; US 7,988,840; US 8,021,614; US 8,282,799; US8,304,230; US 8,579,117; US 2006/0134599; US 2007/0160503; US 20050282293; US 2006/0121624; US 2005/0266433; US 2007/0026381; US 2007/0026414; US 2007/0026417; US 2007/0026415; US 2007/0026413; US 2007/0099207; US 2007/0196820; US 2007/0059680; US 2007/0059718; US 2007/005916; US 2007/0059774; US 2007/0059781; US 2007/0059719; US 2006/0223178; US 2008/0124721; US 2008/0090239; US 2008/0113358; and WO2012094642. Of the various references describing the making and use of devices, US 7,150,812 provides particularly good guidance and 7,735,652 is of particular interest with respect to microfluidic devices for separations performed on samples with cells found in blood (in this regard, see also US 2007/0160503).

A device can be made using any of the materials from which micro- and nano-scale fluid handling devices are typically fabricated, including silicon, glasses, plastics, and hybrid materials. A diverse range of thermoplastic materials suitable for microfluidic fabrication is available, offering a wide selection of mechanical and chemical properties that can be leveraged and further tailored for specific applications.

Techniques for making devices include Replica molding, Softlithography with PDMS, Thermoset polyester, Embossing, Injection Molding, Laser Ablation and combinations thereof. Further details can be found in "Disposable microfluidic devices: fabrication, function and application" by Fiorini, et al. (BioTechniques 38:429-446 (March 2005)), which is hereby incorporated by reference herein in its entirety. The book "Lab on a Chip Technology" edited by Keith E. Herold and Avraham Rasooly, Caister Academic Press Norfolk UK (2009) is another resource for methods of fabrication.

High-throughput embossing methods such as reel-to-reel processing of thermoplastics is an attractive method for industrial microfluidic chip production. The use of single chip hot embossing can be a cost-effective technique for realizing high-quality microfluidic devices during the prototyping stage. Methods for the replication of microscale features in two thermoplastics, polymethylmethacrylate (PMMA) and/or polycarbonate (PC), are described in "Microfluidic device fabrication by thermoplastic hot-embossing" by Yang, etal. (MethodsMol. Biol. 949: 115-23 (2013)).

The flow channel can be constructed using two or more pieces which, when assembled, form a closed cavity (preferably one having orifices for adding or withdrawing fluids) having the obstacles disposed within it. The obstacles can be fabricated on one or more pieces that are assembled to form the flow channel, or they can be fabricated in the form of an insert that is sandwiched between two or more pieces that define the boundaries of the flow channel.

The obstacles may be solid bodies that extend across the flow channel, in some cases from one face of the flow channel to an opposite face of the flow channel. Where an obstacle is integral with (or an extension of) one of the faces of the flow channel at one end of the obstacle, the other end of the obstacle can be sealed to or pressed against the opposite face of the flow channel. A small space (preferably too small to accommodate any particles of interest for an intended use) is tolerable between one end of an obstacle and a face of the flow channel, provided the space does not adversely affect the structural stability of the obstacle or the relevant flow properties of the device.

The number of obstacles present should be sufficient to realize the particle-separating properties of the arrays. The obstacles can generally be organized into rows and columns (Note: Use of the term "rows and columns" does not mean or imply that the rows and columns are perpendicular to one another). Obstacles that are generally aligned in a direction transverse to fluid flow in the flow channel can be referred to as obstacles in a column. Obstacles adjacent to one another in a column may define a gap through which fluid flows.

Obstacles in adjacent columns can be offset from one another by a degree characterized by a tilt angle, designated ε (epsilon). Thus, for several columns adjacent to one another *(i.e.,* several columns of obstacles that are passed consecutively by fluid flow in a single direction generally transverse to the columns), corresponding obstacles in the columns can be offset from one another such that the corresponding obstacles form a row of obstacles that extends at the angle ε relative to the direction of fluid flow past the columns. The tilt angle can be selected and the columns can be spaced apart from each other such that 1/ε (when expressed in radians) is an integer, and the columns of obstacles repeat periodically. The obstacles in a single column can also be offset from one another by the same or a different tilt angle. By way of example, the rows and columns can be arranged at an angle of 90 degrees with respect to one another, with both the rows and the columns tilted, relative to the direction of bulk fluid flow through the flow channel, at the same angle of ε.

Surfaces can be coated to modify their properties and polymeric materials employed to fabricate devices, can be modified in many ways. In some cases, functional groups such as amines or carboxylic acids that are either in the native polymer or added by means of wet chemistry or plasma treatment are used to crosslink proteins or other molecules. DNA can be attached to COC and PMMA substrates using surface amine groups. Surfactants such as Pluronic^{®} can be used to make surfaces hydrophilic and protein repellant by adding Pluronic^{®} to PDMS formulations. In some cases, a layer of PMMA is spin coated on a device, *e.g.,* microfluidic chip and PMMA is "doped" with hydroxypropyl cellulose to vary its contact angle.

To reduce non-specific adsorption of cells or compounds, e.g., released by lysed cells or found in biological samples, onto the channel walls, one or more walls may be chemically modified to be non-adherent or repulsive. The walls may be coated with a thin film coating *(e.g.,* a monolayer) of commercial non-stick reagents, such as those used to form hydrogels. Additional examples of chemical species that may be used to modify the channel walls include oligoethylene glycols, fluorinated polymers, organosilanes, thiols, poly-ethylene glycol, hyaluronic acid, bovine serum albumin, poly-vinyl alcohol, mucin, poly-HEMA, methacrylated PEG, and agarose. Charged polymers may also be employed to repel oppositely charged species. The type of chemical species used for repulsion and the method of attachment to the channel walls can depend on the nature of the species being repelled and the nature of the walls and the species being attached. Such surface modification techniques are well known in the art. The walls may be functionalized before or after the device is assembled.

### III. CAR T Cells

Methods for making and using CAR T cells are well known in the art. Procedures have been described in, for example, US 9,629,877; US 9,328,156; US 8,906,682; US 2017/0224789; US 2017/0166866; US 2017/0137515; US 2016/0361360; US 2016/0081314;US 2015/0299317; and US 2015/0024482; each of which is incorporated by reference herein in its entirety.

### IV. Separation Processes that Use DLD

The DLD devices described herein can be used to purify cells, cellular fragments, cell adducts, or nucleic acids. As discussed herein, these devices can also be used to separate a cell population of interest from a plurality of other cells. Separation and purification of blood components using devices can be found, for example, in US Publication No. US2016/0139012, the teaching of which is incorporated by reference herein in its entirety. A brief discussion of a few illustrative separations is provided below.

### A. Viable Cells

In one embodiment devices are used in procedures designed to separate a viable cell from a nonviable cell. The term "viable cell" refers to a cell that is capable of growth, is actively dividing, is capable of reproduction, or the like. In instances where a viable cell has a size that is greater than a nonviable cell, DLD devices can be designed to comprise a critical size that is greater than a predetermined size of the nonviable cell and less than a predetermined size of the viable cell. The critical size may be as little as 1.1 fold greater than (or less than) the predetermined size of the nonviable cell but generally, larger degrees (or smaller) are preferred, *e.g.,* about 1.2 fold - 2 fold, and preferably 3-10 fold.

### B. Adherent Cells

In another embodiment, DLD devices can be used to in procedures to separate adherent cells. The term "adherent cell" as used herein refers to a cell capable of adhering to a surface. Adherent cells include immortalized cells used in cell culturing and can be derived from mammalian hosts. In some instances, the adherent cell may be trypsinized prior to purification. Examples of adherent cells include MRC-5 cells; HeLa cells; Vero cells; NIH 3T3 cells; L929 cells; Sf21 cells; Sf9 cells; A549 cells; A9 cells; AtT-20 cells; BALB/3T3 cells; BHK-21 cells; BHL-100 cells; BT cells; Caco-2 cells; Chang cells; Clone 9 cells; Clone M-3 cells; COS-1 cells; COS-3 cells; COS-7 cells; CRFK cells; CV-1 cells; D-17 cells; Daudi cells; GH1 cells; GH3 cells; HaK cells; HCT-15 cells; HL-60 cells; HT-1080 cells; HT-29 cells; HUVEC cells; I-10 cells; IM-9 cells; JEG-2 cells; Jensen cells; Jurkat cells; K-562 cells; KB cells; KG-1 cells; L2 cells; LLC-WRC 256 cells; McCoy cells; MCF7 cells; WI-38 cells; WISH cells; XC cells; Y-1 cells; CHO cells; Raw 264.7; BHK-21 cells; HEK 293 cells to include 293A, 293T and the like; HEP G2 cells; BAE-1 cells; SH-SY5Y cells; and any derivative thereof to include engineered and recombinant strains.

In some embodiments, procedures may involve separating cells from a diluent such as growth media, which may provide for the efficient maintenance of a culture of the adherent cells. For example, a culture of adherent cells in a growth medium can be exchanged into a transfection media comprising transfection reagents, into a second growth medium designed to elicit change within the adherent cell such as differentiation of a stem cell, or into sequential wash buffers designed to remove compounds from the culture.

In a particularly preferred procedure, adherent cells are purified through association with one or more carriers that bind in a way that promotes DLD separation. The carriers may be of the type described herein and binding may stabilize and/or activate the cells. A carrier will typically be in the rage of 1-1000 µm but may sometimes also be outside of this range.

The association between a carrier and a cell should produce a complex of increased size relative to other material not associated with the carrier. Depending of the particular size of the cells and carriers and the number of cells and carriers present, a complex may be anywhere from a few percent larger than the uncomplexed cell to many times the size of the uncomplexed cell. In order to facilitate separations, an increase of at least 20% is desirable with higher percentages (50; 100; 1000 or more) being preferred.

### C. Activated Cells

The DLD devices can also be used in procedures for separating an activated cell or a cell capable of activation, from a plurality of other cells. The cells undergoing activation may be grown on a large scale but, in a preferred embodiment, the cells are derived from a single patient and DLD is performed within at least few hours after collection. The terms "activated cell" or "cell capable of activation" refers to a cell that has been, or can be activated, respectively, through association, incubation, or contact with a cell activator. Examples of cells capable of activation can include cells that play a role in the immune or inflammatory response such as: T cells, B cells; regulatory T cells, macrophages, dendritic cells, granulocytes, innate lymphoid cells, megakaryocytes, natural killer cells, thrombocytes, synoviocytes, and the like; cells that play a role in metabolism, such as beta cells, liver cells, and pancreatic cells; and recombinant cells capable of inducible protein expression such as DE3 lysogenized *E. coli* cells, yeast cells, plant cells, etc.

Typically, one or more carriers will have the activator on their surface. Examples of cell activators include proteins, antibodies, cytokines, CD3, CD28, antigens against a specific protein, helper T cells, receptors, and glycoproteins; hormones such as insulin, glucagon and the like; IPTG, lactose, allolactose, lipids, glycosides, terpenes, steroids, and alkaloids. The activatable cell should be at least partially associated with carriers through interaction between the activatable cell and cell activator on the surface of the carriers. The complexes formed may be just few percent larger than the uncomplexed cell or many times the size of the uncomplexed cell. In order to facilitate separations, an increase of at least 20% is desirable with higher percentages (40, 50, 100, 1000 or more) being preferred.

### D. Separating Cells from Toxic Material

DLD can also be used in purifications designed to remove compounds that may be toxic to a cell or to keep the cells free from contamination by a toxic compound. Examples include an antibiotic, a cryopreservative, an antifungal, a toxic metabolite, sodium azide, a metal ion, a metal ion chelator, an endotoxin, a plasticizer, a pesticide, and any combination thereof. The device can be used to remove toxic compounds from cells to ensure consistent production of material from the cells. In some instances, the cell can be a log phase cell. The term "log phase cell" refers to an actively dividing cell at a stage of growth characterized by exponential logarithmic growth. In log phase, a cell population can double at a constant rate such that plotting the natural logarithm of cell number against time produces a straight line.

The ability to separate toxic material may be important for a wide variety of cells including: bacterial strains such as BL21, Tuner, Origami, Origami B, Rosetta, C41, C43, DH5α, DH10β, or XL1Blue; yeast strains such as those of genera Saccharomyces, Pichia, Kluyveromyces, Hansenula and Yarrowia; algae; and mammalian cell cultures, including cultures of MRC-5 cells; HeLa cells; Vero cells; NIH 3T3 cells; L929 cells; Sf21 cells; Sf9 cells; A549 cells; A9 cells; AtT-20 cells; BALB/3T3 cells; BHK-21 cells; BHL-100 cells; BT cells; Caco-2 cells; Chang cells; Clone 9 cells; Clone M-3 cells; COS-1 cells; COS-3 cells; COS-7 cells; CRFK cells; CV-1 cells; D-17 cells; Daudi cells; GH1 cells; GH3 cells; HaK cells; HCT-15 cells; HL-60 cells; HT-1080 cells; HT-29 cells; HUVEC cells; I-10 cells; IM-9 cells; JEG-2 cells; Jensen cells; Jurkat cells; K-562 cells; KB cells; KG-1 cells; L2 cells; LLC-WRC 256 cells; McCoy cells; MCF7 cells; WI-38 cells; WISH cells; XC cells; Y-1 cells; CHO cells; Raw 264.7; BHK-21 cells; HEK 293 cells to include 293A, 293T and the like; HEP G2 cells; BAE-1 cells; SH-SY5Y cells; stem cells and any derivative thereof to include engineered and recombinant strains.

### E. Purification of Material Secreted from Cells

The DLD devices may also be used in the purification of material secreted from a cell. Examples of such secreted materials includes proteins, peptides, enzymes, antibodies, fuel, biofuels such as those derived from algae, polymers, small molecules such as simple organic molecules, complex organic molecules, drugs and pro-drugs, carbohydrates and any combination thereof. Secreted products can include therapeutically useful proteins such as insulin, Imatinib, T cells, T cell receptors, Fc fusion proteins, anticoagulants, blood factors, bone morphogenetic proteins, engineered protein scaffolds, enzymes, growth factors, hormones, interferons, interleukins, and thrombolytics.

FIG. 15 is a schematic depicting the use of DLD in the purification of secreted products. In some instances, the cells may be in an aqueous suspension of buffer, growth medium, or the like, such that the cell secretes product into the suspension. Examples of such secreted products include proteins, peptides, enzymes, antibodies, fuel, biofuels such as those derived from algae, polymers, small molecules such as simple organic molecules, complex organic molecules, drugs and pro-drugs, carbohydrates and any combination thereof. Secreted products can include therapeutically useful proteins such as insulin, Imatinib, T cells, T cell receptors, Fc fusion proteins, anticoagulants, blood factors, bone morphogenetic proteins, engineered protein scaffolds, enzymes, growth factors, hormones, interferons, interleukins, and thrombolytics.

Purification might be carried out, for example, in situations where cells have a predetermined size that is greater than a predetermined size of the secreted compound, where the predetermined size of the cell is greater than or equal to a critical size, and the predetermined size of the secreted compound is less than the critical size. In such a configuration, when applied to a DLD device, the cells can be deflected in a first direction while the secreted compound can be deflected in a second direction, thereby separating the secreted compound from the cell. Also, a secreted protein may be captured by a large carrier that binds in a way that promotes DLD separation. DLD may then be performed and the carrier-protein complex may then be treated to further purify, or release, the protein.

Such processes can be carried out in an iterative fashion such that a population of separated particles can be continuously looped back into a device for further separation. In this regard, FIGS. 16 and 17 are schematics of an iterative process in which separated cells are looped back into the DLD device after separation. In some instances, the cells may be looped from a first device into a second, different device with obstacles comprising different critical sizes. Such a system can allow systematic separation of a plurality of size ranges by manipulating the range of critical sizes. In other instances, cells may be looped back to the same device used previously to separate the isolated particles. This system can be advantageous for continuous purification of actively dividing cells or compounds being actively expressed. For example, such a method could be combined with the method of purifying the secreted product to both collect the secreted product from one flow stream and the cell producing the secreted product from another flow stream. Because the cells can continuously produce the secreted product, the purified cells can be reapplied to the device to continuously collect the secreted product from the cells.

### F. Purity and Yields

The purity, yields and viability of cells produced by the DLD methods discussed herein will vary based on a number of factors including the nature of the starting material, the exact procedure employed and the characteristics of the DLD device. Preferably, purifications, yields and viabilities of at least 60% should be obtained with, higher percentages, at least 70, 80 or 90% being more preferred. In a preferred embodiment, methods may be used to isolate leukocytes from whole blood, apheresis products or leukapheresis products with at least 70% purity, yield and viability with higher percentages (at least 80%, 85%, or 90%) being preferred.

### V. Technological Background

Without being held to any particular theory, a general discussion of some technical aspects of microfluidics may help in understanding factors that affect separations carried out in this field. A variety of microfabricated sieving matrices have been disclosed for separating particles (Chou, et. al., Proc. Natl. Acad. Sci. 96:13762 (1999); Han, et al., Science 288:1026 (2000); Huang, et al., Nat. Biotechnol. 20:1048 (2002); Turner et al., Phys. Rev. Lett. 88(12):128103 (2002); Huang, et al., Phys. Rev. Lett. 89:178301 (2002); U.S. Pat. No. 5,427,663; U.S. Pat. No. 7,150,812; U.S. Pat. No. 6,881,317). Bump array (also known as "obstacle array") devices have been described, and their basic operation is explained, for example in U.S. Pat. No. 7,150,812. A bump array operates essentially by segregating particles passing through an array (generally, a periodically-ordered array) of obstacles, with segregation occurring between particles that follow an "array direction" that is offset from the direction of bulk fluid flow or from the direction of an applied field (U.S. 7,150,812).

### A. Bump Arrays

In some arrays, the geometry of adjacent obstacles is such that the portions of the obstacles defining the gap are symmetrical about the axis of the gap that extends in the direction of bulk fluid flow. The velocity or volumetric profile of fluid flow through such gaps is approximately parabolic across the gap, with fluid velocity and flux being zero at the surface of each obstacle defining the gap (assuming no-slip flow conditions) and reaching a maximum value at the center point of the gap. The profile being parabolic, a fluid layer of a given width adjacent to one of the obstacles defining the gap contains an equal proportion of fluid flux as a fluid layer of the same width adjacent to the other obstacle that defines the gap, meaning that the critical size of particles that are 'bumped' during passage through the gap is equal regardless of which obstacle the particle travels near.

In some cases, particle size-segregating performance of an obstacle array can be improved by shaping and disposing the obstacles such that the portions of adjacent obstacles that deflect fluid flow into a gap between obstacles are not symmetrical about the axis of the gap that extends in the direction of bulk fluid flow. Such lack of flow symmetry into the gap can lead to a non-symmetrical fluid flow profile within the gap. Concentration of fluid flow toward one side of a gap *(i.e.,* a consequence of the non-symmetrical fluid flow profile through the gap) can reduce the critical size of particles that are induced to travel in the array direction, rather than in the direction of bulk fluid flow. This is because the non-symmetry of the flow profile causes differences between the width of the flow layer adjacent to one obstacle that contains a selected proportion of fluid flux through the gap and the width of the flow layer that contains the same proportion of fluid flux and that is adjacent to the other obstacle that defines the gap. The different widths of the fluid layers adjacent to obstacles define a gap that exhibits two different critical particle sizes. A particle traversing the gap can be bumped *(i.e.,* travel in the array direction, rather than the bulk fluid flow direction) if it exceeds the critical size of the fluid layer in which it is carried. Thus, it is possible for a particle traversing a gap having a non-symmetrical flow profile to be bumped if the particle travels in the fluid layer adjacent to one obstacle, but to be not-bumped if it travels in the fluid layer adjacent to the other obstacle defining the gap.

In another aspect, decreasing the roundness of edges of obstacles that define gaps can improve the particle size-segregating performance of an obstacle array. By way of example, arrays of obstacles having a triangular cross-section with sharp vertices can exhibit a lower critical particle size than do arrays of identically-sized and -spaced triangular obstacles having rounded vertices.

Thus, by sharpening the edges of obstacles defining gaps in an obstacle array, the critical size of particles deflected in the array direction under the influence of bulk fluid flow can be decreased without necessarily reducing the size of the obstacles. Conversely, obstacles having sharper edges can be spaced farther apart than, but still yield particle segregation properties equivalent to, identically-sized obstacles having less sharp edges.

### B. Fractionation Range

Objects separated by size on microfluidic include cells, biomolecules, inorganic beads, and other objects. Typical sizes fractionated range from 100 nanometers to 50 micrometers. However, larger and smaller particles may also sometimes be fractionated.

### C. Volumes

Depending on design, a device or combination of devices might be used to process between about 10 µl to at least 500 µl of sample, between about 500 µl and about 40 mL of sample, between about 500 µl and about 20 mL of sample, between about 20 mL of sample and about 200 mL of sample, between about 40 mL of sample and about 200 mL of sample, or at least 200 mL of sample.

### D. Channels

A device can comprise one or multiple channels with one or more inlets and one or more outlets. Inlets may be used for sample or crude *(i.e.*, unpurified) fluid compositions, for buffers or to introduce reagents. Outlets may be used for collecting product or may be used as an outlet for waste. Channels may be about 0.5 to 100 mm in width and about 2-200 mm long but different widths and lengths are also possible. Depth may be 1- 1000 µm and there may be anywhere from 1 to 100 channels or more present. Volumes may vary over a very wide range from a few µl to many ml and devices may have a plurality of zones (stages, or sections) with different configurations of obstacles.

### E. Gap Size (Edge-to-Edge Distance Between Posts or Obstacles)

Gap size in an array of obstacles (edge-to-edge distance between posts or obstacles) can vary from about a few (*e.g*., 1-500) micrometers or be more than a millimeter. Obstacles may, in some embodiments have a diameter of 1-3000 micrometers and may have a variety of shapes (round, triangular, teardrop shaped, diamond shaped, square, rectangular etc.). A first row of posts can be located close to *(e.g.* within 5 µm) the inlet or be more than 1 mm away.

### F. Stackable chips

A device can include a plurality of stackable chips. A device can comprise about 1 - 50 chips. In some instances, a device may have a plurality of chips placed in series or in parallel or both.

### Examples

The following example is intended to illustrate, but not limit the invention.

This study focuses on apheresis samples, which are integral to CAR-T-cell manufacture. The inherent variability associated with donor health, disease status and prior chemotherapy all impact the quality of the leukapheresis collection, and likely the efficacy of various steps in the manufacturing protocols (Levine, et al., Mol. Therapy: Meth. Clin. Dev. 4:92-101 (2017)). To stress test the automated DLD leukocyte enrichment, residual leukocytes (LRS chamber fractions) were collected from plateletpheresis donations which generally have near normal erythrocyte counts, 10-20-fold higher lymphocytes and monocytes and almost no granulocytes. They also have ~10-fold higher platelet counts, as compared to normal peripheral blood.

12 donors were processed and yields were compared of major blood cell types and processivity by DLD versus Ficoll-Hypaque density gradient centrifugation, a "gold standard." 4 of these donors were also assessed for "T-cell expansion capacity" over a 15-day period. Each donor sample was processed by both DLD, and Ficoll, and for the 4 donors studied for T-cell expansion capacity the sample was processed using direct magnetic extraction.

### Materials and Methods

**Microchip design and fabrication:** The DLD array used in this study consisted of a single-zone, mirrored, diamond post design (see D'Silva, J., "Throughout Microfluidic Capture of Rare Cells from Large Volumes of Blood;" A Dissertation Presented to the Faculty of Princeton University in Candidacy for the Degree of Doctor of Philosophy (2016)). There were 14 parallel arrays per chip resulting in a 14-lane DLD device (Fig. 1D). The device was designed with a 16 µm gap between posts and a 1/42 tilt, resulting in a critical diameter of ~ 4 µm. The plastic DLD device was generated using a process called soft-embossing. First, a silicon (Si) master for the plastic DLD microchip was made using standard photolithographic and deep reactive ion etching techniques (Princeton University, PRISM). The features on the silicon master were then transferred to a soft elastomeric mold (Edge Embossing, Medford, MA) by casting and curing the elastomer over the Si features. The elastomer was peeled off to create a reusable, negative imprint of the silicon master. A plastic blank sheet was placed between the elastomer molds, and then using a combination of pressure and temperature, the plastic was extruded into the features (wells) of the soft-elastomer negative mold, replicating the positive features and depth of the original silicon master. The soft tool was then peeled off from the plastic device, producing a flat piece of plastic surface-embossed to a depth ~100 µm with a pattern of flow channels and trenches around an array of microposts (Fig. 1D, inset). Ports were created for fluidic access to the Input and Output ends of the microchip. After cleaning by sonication, the device was lidded with a heat-sensitive, hydrophilic adhesive (ARFlow Adhesives Research, Glen Rock, PA). The overall chip was 40 × 75 mm, and 1 mm thick - smaller than the size of a credit card.

**DLD Microchip operation:** The microfluidic device was assembled inside an optically transparent and pressure resistant manifold with fluidic connections. Fluids were driven through the DLD microchip using a constant pneumatic pressure controller (MFCS-EZ, Fluigent, Lowell, MA). Two separate pressure controls were used, one for buffer and one for sample. The flow path for the buffer line included tubing connecting a buffer reservoir (60 mL syringe), an in-line degasser (Biotech DEGASi, Minneapolis, MN) and the buffer inlet port of the manifold. The flow path for the sample included tubing connecting a sample reservoir (20 mL syringe), a 20µm PureFlow nylon filter of 25mm diameter (Clear Solutions, Inc. San Clemente, CA) to retain aggregates larger than the microchips nominal gap size (16 µm), and the sample inlet port on the manifold. The outlet ports of the manifold were connected by tubing to collection reservoirs for the waste and product fractions.

The microchips, filter and tubing were primed and blocked for 15 min with running buffer before the sample was loaded. The DLD setup was primed by loading running buffer into the buffer reservoir (60 mL syringe) and then pressurizing; fluid then passed through the tubing and into the manifold "Buffer in" port (Fig. 1). Air in the manifold port was vented via another port on that inlet, and then that port was sealed. The buffer was then driven through the microchip and out both the product and waste outlets, evacuating all air in the micropost array. At the same time, buffer was back flushed up through the "Sample IN" port on the manifold and through the in-line filter, flushing any air. This priming step took ~5 min of hands-on time, and removed all air from the microchip, manifold and tubing. Following the prime step, buffer continued to flush the setup for an additional 15 minutes to block all the interior surfaces; this step was automated and did not require hands-on time.

Following the block step, the system was depressurized, and sample was loaded into the sample container (20 mL syringe). The sample (see below) was diluted 1-part sample to 4 parts running buffer (0.2x) prior to loading on the DLD. The buffer source was re-pressurized first, then the sample source, resulting in both buffer and sample entering their respective ports on the manifold and microchip and flowing through the microchip in parallel (see separation mode, Fig. 1 Ai). Once the sample was loaded and at running pressure, the system automatically processed the entire sample volume. Both product and waste fractions were collected in pre-weighed sterile conical 50mL tubes and weighed after the collection to determine the volumes collected.

**Buffer systems.** Three different EDTA free buffer formulations were tested on the DLD: 0.5% F127 (Pluronic F-127, Sigma Aldrich, St. Louis, MO) in phosphate-buffered saline [Ca⁺⁺/Mg⁺⁺ free) (Quality biological, Gaithersburg, MD), 1% Bovine Serum Albumin (BSA) (Affymetrix, Santa Clara, CA) in phosphate-buffered saline [Ca⁺⁺/Mg⁺⁺ free], and an isotonic Elutriation Buffer (EB) composed of 50% Plasmalyte A (Baxter, Deerfield, IL) and 50% of a mixture containing 1.0% BSA (Affymetrix, Santa Clara, CA) 1.0mM N-Acetyl-Cysteine, 2% Dextrose and 0.45% NaCl (all from Sigma-Aldrich, St. Louis, MO). The buffers were prepared fresh each day, and were sterile-filtered through a 0.2 µm filter flask prior to use on the DLD. All samples in the expansion group were processed using the isotonic elutriation buffer to best align with current CAR-T-cell manufacturing approaches, even though better DLD performance has been established with the addition of poloxamer (Johnson, et al., Cancer Cell Res. 27:38-58 (2017)).

**Biological Samples.** Leucoreduction System (LRS) chamber samples from plateletpheresis donations of normal screened donors using a Trima system (Terumo, Tokyo, Japan) were obtained from the local blood bank. Cell counts were done at the time of collection by the blood bank. Counts were verified in our lab, using a Beckman Coulter AcT2 Diff2 clinical blood analyzer, and ranged between 76-313.3×10³ WBC/µL and 0.8-4.87 × 10⁶ platelets/µL. All samples were kept overnight at room temperature on an orbital shaker (Biocotek, China), and then processed the following day (~24 hours later) to mimic overnight shipment. Each donor sample was processed by both DLD, and Ficoll, and for the 4 donors used for T-cell expansion and immunophenotypic studies the sample was also processed using direct magnetic extraction.

**Ficoll-Hypaque.** Peripheral blood mononuclear cells (PBMCs) were obtained by diluting the LRS sample to 0.5X in RPMI (Sigma-Aldrich. St Louis, MO), layered on top of an equal volume of Ficoll-Hypaque (GE, Pittsburgh, PA) in a 50mL conical tube, and centrifuged for 35 min with a free-swinging rotor, and no brake, at 400xg. After centrifugation, the top layer was discarded and the interface PBMC fraction transferred to a new 50mL tube and brought up to 20mL of RPMI. PBMCs were washed by centrifugation for 10 min at 400xg, the supernatant discarded and the pellet resuspended with 20 mL of RPMI and washed again at 200xg for 10min. The supernatant was removed and the pellet resuspended in full media containing RPMI-1640 + 10% Fetal Bovine Serum (FBS) (Sigma-Aldrich, St. Louis, MO) plus penicillin 100 units/mL and streptomycin 100µg/mL antibiotics (Thermo-Fisher, Waltham, MA).

**Cell Isolation, Counting, and Immunofluorescence Staining.** Prior to and after isolation using the methods described above, the cell counts of the resulting products were determined using a blood cell analyzer (Beckman-Coulter AcT2 Diff2). Once in culture, and after activation, cell counts were determined using the Scepter^{™} 2.0 hand-held cell counter (Millipore, Billerica, MA) and by absolute counting using flow cytometry. Cells from the input, product and waste fractions were then loaded onto poly-lysine-coated slides for 10 min and then fixed for 15 min in 4% p-formaldehyde + 0.5 % Triton X-100 in PBS, before washing 3 times in PBS by centrifugation. Slides were incubated with the conjugated primary antibodies CD41-A647 and CD41-FITC (both from BioLegend San Diego, CA) for 60 min in the dark and washed three times with PBS before mounting in slow-fade mounting media containing the DNA stain DAPI (Thermo-Fisher, Waltham, MA). Slides were viewed with an Etaluma^{™} Lumascope 620 fluorescence inverted microscope (Carlsbad, CA). Antibodies (mAb) conjugated to fluorochromes were obtained from BioLegend (San Diego, CA): CD25-PE, CD25-APC, CD95-FITC, CD45RA-BV605, CD45RO-PECy7, CD197/CCR7 PE, CD279-PE, CD28 PE-Cy5, CD45-PerCP, CD3-FITC, CD3-BV421, CD4-AF700, CD8-APC-AF780, CD61-FITC, CD41-FITC, CD45-Alexa647. Viability of the WBCs obtained by DLD and PBMCs purified by Ficoll-Hypaque was determined by Trypan blue exclusion.

**Activation and Magnetic Separation.** For T-cell stimulations in expansion group, DLD, Ficoll and LRS product were diluted to 1 × 10⁷ T cells/mL then activated with CD3/CD28 washed and equilibrated anti-CD3/CD28 conjugated magnetic beads (5.0µm) (Thermo-Fisher, Waltham, MA) at a ratio of 3.2:1 beads per cell for 60 min, and then the activated T cells were separated by a magnetic depletion for 5 min. Unbound cells were removed, and the bead-bound cells were cultured further in full media (below). In the direct magnet protocol, 0.5mL of LRS sample (same donor as was processed via DLD or Ficoll) was incubated with immunomagnetic CD3/CD28 beads for one hour. The mixture was then placed against a magnet for 5 minutes to capture the T cells. The magnetic bead-bound cells (activated cells) were removed and then diluted to 0.5×10⁶/mL as above for culture in full media.

After three days in culture, recombinant human IL-2 (BioLegend, San Diego, CA) was added at 200 IU/mL to wells. Following cell culture for up to 15 days, beads were removed from cells and cells counted at each time point. To remove beads, the cells in the well were resuspended by passing the cells through a 5-mL pipette for 10 times. Next, the cell suspension was passed throughout a 1 mL pipette 40 times followed by vigorous pipetting using a 200 µL tip for 1 min. Then the cell suspension was placed on the side of a magnet for 5 min and the nonmagnetic fraction was transferred to a fresh tube and counted. The number of cells in the culture wells was determined using a Scepter hand-held cell counter and by flow cytometry.

**Cell Culture and Cell Activation.** For each of the T-cell preparations put into cell culture, in addition to the stimulated cells described above, unstimulated cells (controls) were adjusted to 0.5×10⁶/mL in complete media (RPMI + 10% FBS + antibiotics) and plated in 6-well plates (Corning, NY) and cultured at 37⁰C, 5% CO₂ in a humidified incubator. Individual wells, for each condition, unstimulated, and stimulated with and stimulated without IL2, were dedicated to each donor at each time point to eliminate any possibility of disruption in expansion due to sampling and the de-beading activity required for reliable counts, particularly at Day 3.

**Flow Cytometry.** No-wash absolute counting by flow cytometry was used for CD3+ cell counts at all time points, Initial day 0 counts used TruCount tubes (BD Biosciences, San Jose, CA) to accurately determine the number of cells recovered and counted. Subsequent days used 25,000 123 beads (Affymetrix, Santa Clara, CA) which were indexed against TruCount tubes as an internal control. 100 µL of a cell suspension was stained with the CD3 FITC, CD25 PE and CD45 PerCP of conjugated antibodies for 30min in the dark in either TruCount tubes or with addition of 25,000 123 beads (Affymetrix, Santa Clara, CA). The cells were then diluted to 250µL of PBS with a final DRAQ5^{™} DNA dye (Thermo-Fisher, Waltham, MA) concentration of 1.0mM. Next, the stained cells were fixed with an additional 250 µL 1.2% p-formaldehyde in PBS overnight prior to acquisition. For absolute count cytometry, a minimum of 25,000 events or 2500 bead events were acquired on a BD FACSCalibur (BD Biosciences, San Jose, CA) using a fluorescence threshold (CD45 PerCP). Phenotypic analysis was also performed at all time points, using a 7-color activation/anergy panel consisting of CD3, CD45RA, CD95, CD279, CD25, CD4, and CD8. At day 15 the panel was modified to create a 9-color panel focused on T central memory cells which added CD45RO PE-Cy7, CD28 PE-Cy5 and substituted CD197/CCR7 PE for CD279/PD1 PE. For multicolor staining, 100µl of a cell suspension was stained as above, and resuspended in 750µL PBS and washed by centrifugation at 400xg and then resuspending in 250µL 1.2% p-formaldehyde and fixed overnight prior to acquiring 20,000 events using forward scatter threshold on a four laser BD FACS Aria II. (BD Biosciences, San Jose, CA). All data analysis was performed using Flowlogic Software (Inivai, Melbourne, Australia).

### RESULTS

### DLD microchip and Ficoll processing of apheresis products

The DLD and Ficoll separation methods were used to process 12 LRS samples obtained from 12 separate normal donors. Of those 12 samples received and processed, 11 samples clustered around a mean of 148.7 × 10³/ µL WBC and 2.52 × 10⁶/µL platelet counts respectively (Fig. 2A, 2B). The 12^{th} sample, with 313.3× 10³/ µL WBC and 4.87 × 10⁶/µL platelet counts can be seen in the scatter plot as a triangle, (Fig. 2A). This sample was sufficiently aggregated at the time of processing that it rapidly clogged the 20µm prefilter and thus did not fully enter the DLD. Microscopic examination of the input sample showed that this sample was full of platelet-WBC aggregates ranging in size from 25-50µm with multiple aggregates observed as large as 250µm in diameter (Fig. 2C, 2D). Further, both WBC and platelet counts were greater than 3 standard deviations above the mean WBC and platelet count. Using the quartile method, this sample was classified as a mild outlier; using the Grubbs test for outliers and an alpha level of 0.05, this sample was also classified as an outlier.²⁰ As a result, this donor was excluded from the study based on extremely high WBC and platelet counts and being too badly agglutinated and damaged.

A representative image of the input material (LRS product diluted to 0.2x) is shown in (Fig. 2A). Typical micrographs of DLD (Fig. 2E) and Ficoll (Fig. 2C) cell products from the same input donor, with significantly lower background platelet levels (CD41-FITC in green) found in the DLD compared to Ficoll. Also shown are the respective cell products, as collected in tubes (Fig. 2 G, H). DLD processing automated the process of removing the WBCs from the RBCs and platelets, generating one tube for product and one for waste, while the Ficoll sample still requires further manual processing to pipet the PMBC layer at the operationally-defined interface of the plasma layer above and Ficoll layer below (Fig. 2H); plus, an additional minimum of two centrifugal washes are required to remove most of the contaminating platelets.

The recovery of WBC, and RBC and platelet depletions of the 11 samples are summarized in Table 2. Mean cell recoveries of PBMC from DLD were -80%, 17% higher than Ficoll (63%), and, after accounting for the number of CD3 cells in both the DLD and magnetic samples, the DLD product was 36% higher than Direct Magnet (44%). Mean platelet depletion via DLD (83%) was superior to both Ficoll (56.5%) and direct magnet (77%). Mean erythrocyte depletion in these 24-hour old samples was 97% for both DLD and Ficoll, and 94% for the direct magnet approach. The average viability of cells obtained by DLD was 96% compared to Ficoll which were 97%.

The average total time taken to process equivalent aliquots of a single sample in a 50mL conical tube via the Ficoll technique was timed at ~90 minutes, with approximately 30 minutes of skilled hands-on time required. Timed runs using our single microchip layer breadboard system processed in much shorter time, 50 minutes and required 25 minutes of hands on time, with approximately 20 minutes being due solely to assembly of fluidics components because of the prototypic nature of the otherwise intervention free device.

### Cell Expansion and Characterization

Following DLD or Ficoll enrichment, cells were activated using CD3/CD28 magnetic beads for 60 minutes at a target of 3.2 beads per CD3+ cell, separated and then counted prior to plating. Due to limited access to a flow cytometer, and concerns regarding potential bead interference in product cell counts, we estimated the T cell count by counting both the input and non-magnetic fraction and getting the number of T cells bound to the magnet by subtraction, using an assumption of a 90% efficient magnetic separation (based on manufacturer reported efficiencies). Accurate T-cell counts were determined post-plating into culture using absolute counts by flow cytometry and by coulter counts x %CD3 positive cells; these counts established that the original magnetic CD3+ cell depletion process was only 44% efficient (Table 2). This meant that original calculations pertaining to a target of 3.2 beads per CD3+ cell were in fact on average 2.3 for both the DLD and Ficoll fractions (fewer beads per T-cell than targeted), and a 5:1 ratio in the direct magnet fraction (significantly more beads per T-cell than targeted), potentially causing the direct magnet fraction to have even higher fold expansion compared to both the DLD and Ficoll arms.

Flow cytometric characterization of the cultures was performed at each time point to assess consistency of cell activation. Changes in CD25 expression of CD3+ cells, as measured on Day 8, for Ficoll, DLD and direct magnet (Fig. 3). IL-2 Receptor positive (CD25) CD3 cells were shown in Blue (CD4+ plots) and Red (CD8+ plots). DLD prepared cells show more consistent phenotypic expression across the 4 donors for CD25, an indicator of response to CD3/CD28 stimulation, as compared to both Ficoll and direct magnet preparations. DLD prepared CD3+ cells had an average 73% response to co-stimulation compared to Ficoll at 51% (both stimulated at 2.3 beads/cell), while the direct magnet fraction, stimulated at a higher 5:1 ratio, had only a 54% response.

Unstimulated controls for Ficoll and DLD show a marked difference, with DLD prepared cells remaining CD25 negative in culture compared to Ficoll (Fig. 9). Interestingly, Donor 37 in the direct magnet fraction did not respond by day 8, but did expand at later time points (also shown in (Fig. 5A)) indicating a potentially delayed response of some samples to the direct magnetic approach.

In addition to evaluating CD25, conversion to a memory cell phenotype was tracked using percentage of CD3+ cells that were CD45RA- and CD25+. The results shown in Fig. 4 indicate a greater percentage of the cultured cells, as generated via DLD, were responsive to co-stimulation compared to cells processed by Ficoll and direct magnetics. Further, the percent of CD3 cells that were CD25- CD45RA- was lowest in the DLD fraction at 12% as compared to 33% and 29% for Ficoll and Direct Magnet respectively, indicating a more complete conversion towards the CD25+ CD45RA- population with the DLD CD3 cells. The standard deviation of the CD45RA-CD25+ population at day 8 for DLD was 10.1% as compared to 24.8% for Ficoll and 53.4% for Direct Magnet.

The fold expansion of the individual cultures was determined at day 3, day 8 and day 15; that data is shown in Figure 5A. The plot shows the expansion of each donor sample, across each method. While the direct magnet approach appears to show higher expansion, the counts are likely significantly affected by the different bead:cell ratios (and corresponding differences in plating density). Regardless, the 4 donors show significant variability in the fold expansion. In addition, the day 15 culture for the direct magnet arm donor #21 became contaminated and had to be discarded, despite having antibiotics present. It is not possible to know if the day 8 expansion data for donor #21 were influenced by the contaminant.

Comparisons between the Ficoll and DLD are valid and much more direct: these cells were plated at the same density and stimulated at the same bead:cell ratio. While the average fold expansion of the DLD cells is not significantly higher than that of the Ficoll cells, the consistency of expansion across the set of 4 donors, and at all days surveyed, is striking. Further the percent of cells in culture that are a central memory phenotype is on average 74% for the DLD arm, contrasted to 47% and 48% respectively for the Ficoll and Direct Magnet arms. Multiplying fold expansion in Fig. 5A by percent yield (table 1) and percent memory (Fig. 5B) shows that, despite the sub optimal comparison with bead:cell ratios, that on average twice as many memory cells were produced from the DLD arm as compared to either Ficoll or Direct Magnet arms.

Figure 6 shows the phenotypic approach to identifying memory cells used in this study, which is designed to eliminate any issues with shed antigens such as CD62L (Mahnke, et al., Eur. J. of Immunol. 43:2797-2809 (2013)). Central memory cells are sequentially gated and then backgated to show the CD3+ T cells are positive for CD45R0+, CD95+, CD28+ and CD197/CCR7+ against all other CD3+ cells in the culture. Using an arbitrary value greater than 50% of the culture as being a central memory phenotype as a conversion metric, the DLD arm showed 100% (4/4) donors achieving central memory conversion with an average of 74% of cells being of memory phenotype, with coefficient of variation across donors of 13%. In contrast, the Ficoll arm showed 50% (2/4) converting with an average of 47% memory cells, and a 29% variation.

The direct magnet arm achieved 33% (1/3) conversion with an average of 48% memory cells and an associated 79% variation.

**Table 2. Comparison of DLD, Ficoll and Direct Magnetic Enrichment**

| | WBC Recovery DLD (n=11) | RBC Depletion | Platelet Depletion |
|---|---|---|---|
| Average | 79.6% | 96.9% | 83.1% |
| STDEV | 13.4% | 1.1% | 12.3% |
| Range | 46.5 - 93.7% | 95.5 - 98.6% | 60.5 - 100.0% |
| Median | 80.1% | 97.0% | 87.6% |
| | Ficoll (n=11) | | |
| Average | 63.5% | 97.1% | 56.5% |
| STDEV | 16.3% | 1.7% | 22.8% |
| Range | 22.4 - 83.7% | 94.1 - 99.9% | 67.0 - 92.1% |
| Median | 65.6% | 97.0% | 52.3% |
| | Direct Magnet (CD3 positive) (n=4) | | |
| Average | 44.0% | 94.1% | 77.6% |
| STDEV | 5.8% | 3.3% | 10.4% |
| Range | 36.8-50.7% | 90.1 - 97.6% | 25.0 - 99.1% |
| Median | 65.6% | 94.5% | 76.0% |

### REFERENCES

1. Vonderheide, R.H., and June, C.H. Engineering T-cells for Cancer: Our Synthetic Future. Immunol. Rev. 2014, 257, 7-13.
2. Fousek, K., and Ahmed, N. The Evolution of T-cell Therapies for Solid Malignancies. Clin. Cancer Res. 2015, 21, 3384-3392.
3. Wang, X and Riviere, I. Clinical Manufacturing of CAR-T-cells: Foundation of a Promising Therapy. Mol. Ther. Oncolytics 2016, 3, 16015.
4. Sadelain, M., Rivière, I. and Riddell, S. Therapeutic T-cell engineering. Nature, 2017, 545, 423-431.
5. National Cell Manufacturing Consortium. Achieving Large-Scale, Cost-Effective, Reproducible Manufacturing of High Quality Cells. A Technology Roadmap to 2025. February 2016.
6. Levine, B.L., Miskin, J., Wonnacott, K., et al. Global Manufacturing of CAR T-cell Therapy. Mol. Therapy: Meth. Clin. Dev. 2017, 4, 92-101.
7. Couzin-Frankel, J. Supply of Promising T-cell Therapy is Strained. Science 2017, 356, 1112.
8. Johnson, L.A., and June, C.H. Driving Gene-engineered T-cell Immunotherapy of Cancer. Cell Res. 2017, 27, 38-58.
9. Hokland, P., and Heron, I. The Isopaque-Ficoll Method Re-evaluated: Selective Loss of Autologous Rosette-forming Lymphocytes During Isolation of Mononuclear Cells from Human Peripheral Blood. Scand. J. Immunol. 1980, 11, 353-356.
10. Stroncek, D.F., Fellowes, V., Pham, C., et al. Counter-flow Elutriation of Clinical Peripheral Blood Mononuclear Cell Concentrates for the Production of Dendritic and T-cell Therapies. J. Transl. Med. 2014, 12, 241.
11. Powell Jr, D.J., Brennan, A.L., Zheng, Z., et al. Efficient Clinical-scale Enrichment of Lymphocytes for Use in Adoptive Immunotherapy Using a Modified Counterflow Centrifugal Elutriation Program. Cytotherapy 2009, 11, 923-935.
12. TerumoBCT. ELUTRA Cell Separation System. Manufacturer recommendations for the Enrichment of Lymphocytes from Apheresis Residues.
13. C.E. Chiche-Lapierre, C.E., Tramalloni, D, Chaput,N. et al. Comparative Analysis of Sepax S-100, COBE 2991, and Manual DMSO Removal Techniques From Cryopreserved Hematopoietic Stem Cell Apheresis Product Cytotherapy 2016 18, 6:S47.
14. Huang, L, Cox, E, Austin, R. Continuous particle separation through deterministic lateral displacement, Science 2004, 304:987-990.
15. Davis, J.A., Inglis, D.W., et al. Deterministic Hydrodynamics: Taking Blood Apart. Proc. Natl.Acad. Sci. USA 2006, 103, 14779-14784.
16. Inglis, D.W., Davis, J.A., Austin, R.H. Critical particle Size for Fractionation by Deterministic Lateral Displacement. Lab Chip 2006, 6, 655-658.
17. Chen, Y, D'Silva, J. Austin, R et al. Microfluidic chemical processing with on-chip washing by deterministic lateral displacement arrays with separator walls. Biomicrofluidics. 2015 9(5): 054105.
18. Shilun, F. Skelley, A., Anwer, A.G., et al. Maximizing Particle Concentration in Deterministic Lateral Displacement Arrays. Biomicrofluidics 2017, 11, 024121.
19. D'Silva, J. Throughout Microfluidic Capture of Rare Cells from Large Volumes of Blood. A Dissertation Presented to the Faculty of Princeton University in Candidacy for the Degree of Doctor of Philosophy. 2016.
20. NIST/SEMATECH e-Handbook of Statistical Methods, 2017, www.itl.nist.gov/div898/ handbook/August
21. Mahnke, Y.D., Brodie, T.M., Sallusto, F., et al. The Who's Who of T-cell Differentiation: Human Memory T-cell Subsets. Eur. J. of Immunol. 2013, 43,2797-2809.
22. Civin, C.I., Ward, T., Skelley, A.M., et al. Automated Leukocyte Processing by Microfluidic Deterministic Lateral Displacement. Cytometry A. 2016, 89, 1073-1083.
23. Trickett, A., and Kwan, Y.L. T-cell Stimulation and Expansion Using Anti-CD3/CD28 Beads. J. Immunol. Meth. 2003, 275, 251-255.
24. Marktkamcham, S., Onlamoon, N., Wang, S., et al. The Effects of Anti-CD3/CD28 Coated Beads and IL-2 on Expanded T-cell for Immunotherapy. Adv. Clin. Exp. Med. 2016, 25, 821-828.
25. Li, Y., and Kurlander, R.J. Comparison of Anti-CD3 and Anti-CD28-coated Beads with Soluble Anti-CD3 for Expanding Human T-cells: Differing Impact on CD8 T-cell Phenotype and Responsiveness. J. Transl. Med. 2010, 8, 104-118.
26. Agrawal S., Ganguly, S., Hjian, P., et al. PDGF Upregulates CLEC-2 to Induce T Regulatory Cells. Oncotarget. 2015, 6, 28621-28632.
27. Zhu, L., Huang, Z., Stålesen, R. et al. Platelets Provoke Distinct Dynamics of Immune Response by Differentially Regulating CD4+ T-cell Proliferation. J. Throm. Haem. 2014, 12, 1156-1165.
28. Koesdjojo, M., Lee, Z., Dosier, C., et al. DLD Microfluidic Purification and Characterization of Intact and Viable Circulating Tumor Cells in Peripheral Blood. AACR Annual Meeting 2016, Abstract #3956.
29. Loutherback, K. "Microfluidic Devices for High Throughput Cell Sorting and Chemical Treatment," A Dissertation Presented to the Faculty of Princeton University 2011.

## Claims

1. An *in vitro* method of separating an activated cell from a plurality of other cells comprising:
a) contacting a crude fluid composition comprising a cell capable of activation and the plurality of other cells with one or more carriers,
wherein at least one carrier comprises a cell activator,
wherein the cell activator is at least partially associated with the cell capable of activation by the cell activator upon or after contact to generate a carrier associated cell complex,
wherein the association of the cell activator with the cell capable of activation by the cell activator at least partially activates the cell capable of activation,
wherein the carrier associated cell complex comprises an increased size relative to cells in the plurality of other cells, and
wherein a size of the carrier associated cell complex is greater than or equal to a critical size, and cells in the plurality of other cells comprise a size less than the critical size;
b) applying the sample to a device,
wherein the device comprises an array of obstacles arranged in rows;
wherein the rows are shifted laterally with respect to one another,
wherein the rows are configured to deflect a particle greater than or equal to the critical size in a first direction and a particle less than the critical size in a second direction; and
c) flowing the sample through the device,
wherein the association of the carrier comprising the cell activator with the cell capable of activation by the cell activator provides for an increase in size of the complex relative to the unbound cell such that the carrier associated cell complex is deflected by the obstacles in the first direction, and the cells in the plurality of other cells are deflected in the second direction,
thereby separating the activated cell from the other cells of the plurality;
d) collecting a fluid composition comprising the separated carrier associated cell complex.

2. The method of claim 1, wherein the cell capable of activation is selected from the group consisting of: a T cell, a B cell, a regulatory T cell, a macrophage, a dendritic cell, a granulocyte, an innate lymphoid cell, a megakaryocyte, a natural killer cell, a thrombocyte, a synoviocyte, a beta cell, a liver cell, a pancreatic cell; a DE3 lysogenized cell, a yeast cell, a plant cell, a stem cell, and a recombinant cell capable of inducible protein expression.

3. The method of claim 1 or 2, wherein the cell activator is a protein.

4. The method of claim 3, wherein the protein is an antibody.

5. The method of claim 1 or 2, wherein the protein is selected from the group consisting of: CD3, CD28, an antigen, a helper T cell, a receptor, a cytokine, a glycoprotein, and any combination thereof.

6. The method of claim 1, wherein the cell activator is selected from the group consisting of insulin, IPTG, lactose, allolactose, a lipid, a glycoside, a terpene, a steroid, an alkaloid, and any combination thereof.

7. The method of any one of claims 1-6, wherein said cell capable of activation has been collected from a patient as part of a crude fluid composition comprising said cell capable of activation and a plurality of other cells, and wherein no more than four hours elapse from the time that the obtaining of the crude fluid composition from the patient is completed until the cell capable of activation is bound to the carrier.

8. The method of claim 1-6, wherein no more than two hours elapse from the time that the obtaining of the crude fluid composition from the patient is completed until the cell capable of activation is bound to the carrier.

9. The method of any one of claims 1-8, wherein the diameters of all of said carriers are at least as large as the cell capable of activation.

10. The method of any one of claims 1-9, wherein the diameters of all of said carriers are at least ten times as large as that of the cell capable of activation.

11. The method of any one of claims 1-9, wherein the diameters of all of said carriers are no more than 25% as large as the cell capable of activation.

12. The method of any one of claims 1-11, further comprising transfecting or transducing the cell capable of activation or the at least partially activated cell with nucleic acids, wherein the nucleic acids are designed to impart on the cells a desired phenotype.

13. The method of any one of claims 1-11, further comprising transfecting or transducing the cell capable of activation or the at least partially activated cell with nucleic acids, wherein the transfection or transduction with the nucleic acids results in cells expressing a chimeric molecule.

14. The method of claim 12 or 13, wherein the cell capable of activation is a T cell.

15. The method of any one of claims 1-14, wherein the diameter of the complex formed between the cell capable of activation and one or more carriers is at least 20% larger than the uncomplexed cells.

## Patentansprüche

1. In-vitro-Verfahren zur Separation einer aktivierten Zelle aus einer Vielzahl von anderen Zellen umfassend:
a) Inkontaktbringen einer Roh-Flüssigkeitszusammensetzung umfassend eine zur Aktivierung fähige Zelle und eine Vielzahl anderer Zellen mit einem oder mehreren Trägern,
wobei mindestens ein Träger einen Zellaktivator umfasst,
wobei der Zellaktivator zumindest teilweise mit der durch den Zellaktivator bei oder nach Kontakt zur Aktivierung fähigen Zelle assoziiert ist, um einen Träger-assoziierten Zellkomplex zu erzeugen,
wobei die Assoziation des Zellaktivators mit der durch den Zellaktivator zur Aktivierung fähigen Zelle die zur Aktivierung fähige Zelle zumindest teilweise aktiviert,
wobei der Träger-assoziierte Zellkomplex eine größere Größe verglichen mit den Zellen in der Vielzahl anderer Zellen aufweist, und
wobei eine Größe des Träger-assoziierten Zellkomplexes größer als oder gleich einer kritischen Größe ist und die Zellen in der Vielzahl anderer Zellen eine Größe kleiner als die kritische Größe aufweisen;
b) Aufbringen der Probe auf eine Vorrichtung,
wobei die Vorrichtung eine Reihe von in Reihen angeordneten Hindernissen umfasst;
wobei die Reihen seitlich gegeneinander verschoben sind,
wobei die Reihen so konfiguriert sind, dass sie ein Teilchen größer als oder gleich der kritischen Größe in einer ersten Richtung und ein Teilchen kleiner als die kritische Größe in einer zweiten Richtung ablenken; und
c) Fließenlassen der Probe durch das Gerät,
wobei die Assoziation des Trägers umfassend den Zellaktivator mit der zur Aktivierung durch den Zellaktivator fähigen Zelle für eine Vergrößerung der Größe des Komplexes verglichen mit der ungebundenen Zelle sorgt, so dass der Träger-assoziierte Zellkomplex durch die Hindernisse in der ersten Richtung abgelenkt wird und die Zellen in der Vielzahl anderer Zellen in der zweiten Richtung abgelenkt werden,
dadurch Separieren der aktivierten Zelle aus den anderen Zellen der Vielzahl;
d) Sammeln einer Flüssigkeitszusammensetzung, die den separierten Träger-assoziierten Zellkomplex umfasst.

2. Verfahren nach Anspruch 1, wobei die zur Aktivierung fähige Zelle ausgewählt ist aus der Gruppe bestehend aus: einer T-Zelle, einer B-Zelle, einer regulatorischen T-Zelle, einem Makrophagen, einer dendritischen Zelle, einem Granulozyten, einer innaten lymphoiden Zelle, einem Megakaryozyten, einer natürlichen Killerzelle, einem Thrombozyten, einem Synoviozyten, einer Beta-Zelle, einer Leberzelle, einer Pankreaszelle, einer lysogenisierten DE3-Zelle, einer Hefezelle, einer Pflanzenzelle, einer Stammzelle und einer rekombinanten, zur induzierbaren Proteinexpression fähigen Zelle.

3. Verfahren nach Anspruch 1 oder 2, wobei der Zellaktivator ein Protein ist.

4. Verfahren nach Anspruch 3, wobei das Protein ein Antikörper ist.

5. Verfahren nach Anspruch 1 oder 2, wobei das Protein ausgewählt ist aus der Gruppe bestehend aus: CD3, CD28, einem Antigen, einer T-Helferzelle, einem Rezeptor, einem Zytokin, einem Glykoprotein und jeglicher Kombination davon.

6. Verfahren nach Anspruch 1, wobei der Zellaktivator ausgewählt ist aus der Gruppe bestehend aus Insulin, IPTG, Laktose, Allolaktose, einem Lipid, einem Glykosid, einem Terpen, einem Steroid, einem Alkaloid und jeglicher Kombination davon.

7. Verfahren nach einem der Ansprüche 1-6, wobei die zur Aktivierung fähige Zelle einem Patienten als Teil einer Roh-Flüssigkeitszusammensetzung umfassend die zur Aktivierung fähige Zelle und eine Vielzahl anderer Zellen entnommen wurde, und wobei nicht mehr als vier Stunden von dem Zeitpunkt an vergehen, zu dem die Gewinnung der Roh-Flüssigkeitszusammensetzung vom Patienten abgeschlossen ist, bis die zur Aktivierung fähige Zelle an den Träger gebunden ist.

8. Verfahren nach Anspruch 1-6, wobei nicht mehr als zwei Stunden von dem Zeitpunkt an vergehen, zu dem die Gewinnung der Roh-Flüssigkeitszusammensetzung vom Patienten abgeschlossen ist, bis die zur Aktivierung fähige Zelle an den Träger gebunden ist.

9. Verfahren nach einem der Ansprüche 1-8, wobei die Durchmesser aller Träger mindestens so groß sind wie die zur Aktivierung fähige Zelle.

10. Verfahren nach einem der Ansprüche 1-9, wobei die Durchmesser aller Träger mindestens zehnmal so groß sind wie der Durchmesser der zur Aktivierung fähigen Zelle.

11. Verfahren nach einem der Ansprüche 1-9, wobei die Durchmesser aller Träger nicht mehr als 25 % so groß sind wie die zur Aktivierung fähige Zelle.

12. Verfahren nach einem der Ansprüche 1-11, ferner umfassend Transfizieren oder Transduzieren der zur Aktivierung fähigen Zelle oder der zumindest teilweise aktivierten Zelle mit Nukleinsäuren, wobei die Nukleinsäuren so ausgestaltet sind, dass sie den Zellen einen gewünschten Phänotyp verleihen.

13. Verfahren nach einem der Ansprüche 1-11, ferner umfassend Transfizieren oder Transduzieren der zur Aktivierung fähigen Zelle oder der zumindest teilweise aktivierten Zelle mit Nukleinsäuren, wobei die Transfektion oder Transduktion mit Nukleinsäuren zu Zellen führt, die ein chimäres Molekül exprimieren.

14. Verfahren nach Anspruch 12 oder 13, wobei die zur Aktivierung fähige Zelle eine T-Zelle ist.

15. Verfahren nach einem der Ansprüche 1-14, wobei der Durchmesser des zwischen der zur Aktivierung fähigen Zelle und einem oder mehreren Trägern gebildeten Komplexes mindestens 20 % größer ist als die unkomplexierten Zellen.

## Revendications

1. Procédé *in vitro* de séparation d'une cellule activée à partir d'une pluralité d'autres cellules comprenant :
a) la mise en contact d'une composition fluidique brute comprenant une cellule capable d'activation et la pluralité d'autres cellules avec un ou plusieurs supports, dans lequel le au moins un support comprend un activateur cellulaire,
dans lequel l'activateur cellulaire est au moins partiellement associé à la cellule capable d'activation par l'activateur cellulaire lors de ou après un contact afin de générer un complexe cellulaire associé à un support,
dans lequel l'association de l'activateur cellulaire avec la cellule capable d'activation par l'activateur cellulaire active au moins partiellement la cellule capable d'activation,
dans lequel le complexe cellulaire associé à un support comprend une taille augmentée par rapport aux cellules dans la pluralité d'autres cellules, et
dans lequel une taille du complexe cellulaire associé à un support est supérieure ou égale à une taille critique, et les cellules dans la pluralité d'autres cellules comprennent une taille inférieure à la taille critique ;
b) l'application de l'échantillon à un dispositif,
dans lequel le dispositif comprend un réseau d'obstacles disposés en rangées ; dans lequel les rangées sont décalées latéralement les unes par rapport aux autres,
dans lequel les rangées sont configurées pour dévier une particule ayant une taille supérieure ou égale à la taille critique dans une première direction et une particule ayant une taille inférieure à la taille critique dans une deuxième direction ; et
c) la circulation de l'échantillon dans le dispositif,
dans lequel l'association du support comprenant l'activateur cellulaire avec la cellule capable d'activation par l'activateur cellulaire entraîne une augmentation de la taille du complexe par rapport à la cellule non liée de sorte que le complexe cellulaire associé à un support soit dévié par les obstacles dans la première direction, et que les cellules dans la pluralité d'autres cellules soient déviées dans la deuxième direction,
en séparant ainsi la cellule activée des autres cellules de la pluralité ;
d) la collecte d'une composition fluidique comprenant le complexe cellulaire associé à un support séparé.

2. Procédé selon la revendication 1, dans lequel la cellule capable d'activation est sélectionnée dans le groupe consistant en : une cellule T, une cellule B, une cellule T régulatrice, un macrophage, une cellule dendritique, un granulocyte, une cellule lymphoïde innée, un mégacaryocyte, une cellule tueuse naturelle, un thrombocyte, un synoviocyte, une cellule bêta, une cellule hépatique, une cellule pancréatique ; une cellule lysogénisée DE3, une cellule de levure, une cellule végétale, une cellule souche, et une cellule recombinante capable d'expression d'une protéine inductible.

3. Procédé selon la revendication 1 ou 2, dans lequel l'activateur cellulaire est une protéine.

4. Procédé selon la revendication 3, dans lequel la protéine est un anticorps.

5. Procédé selon la revendication 1 ou 2, dans lequel la protéine est sélectionnée dans le groupe consistant en : CD3, CD28, un antigène, une cellule T auxiliaire, un récepteur, une cytokine, une glycoprotéine, et une quelconque combinaison de ceux-ci.

6. Procédé selon la revendication 1, dans lequel l'activateur cellulaire est sélectionné dans le groupe consistant en l'insuline, l'IPTG, le lactose, l'allolactose, un lipide, un glycoside, un terpène, un stéroïde, un alcaloïde, et une quelconque combinaison de ceux-ci.

7. Procédé selon l'une quelconque des revendications 1-6, dans lequel ladite cellule capable d'activation a été collectée chez un patient en tant qu'élément d'une composition fluidique brute comprenant ladite cellule capable d'activation et une pluralité d'autres cellules, et dans lequel il ne s'écoule pas plus de quatre heures entre le moment où la composition fluidique brute est obtenue à partir du patient et la liaison de la cellule capable d'activation au support.

8. Procédé selon la revendication 1-6, dans lequel il ne s'écoule pas plus de deux heures entre le moment où la composition fluidique brute est obtenue chez le patient et la liaison de la cellule capable d'activation au support.

9. Procédé selon l'une quelconque des revendications 1-8, dans lequel les diamètres de l'ensemble desdits supports sont au moins aussi grands que celui de la cellule capable d'activation.

10. Procédé selon l'une quelconque des revendications 1-9, dans lequel les diamètres de l'ensemble desdits supports sont au moins dix fois plus grands que celui de la cellule capable d'activation.

11. Procédé selon l'une quelconque des revendications 1-9, dans lequel les diamètres de l'ensemble desdits supports sont au maximum 25 % plus grands que celui de la cellule capable d'activation.

12. Procédé selon l'une quelconque des revendications 1-11, comprenant en outre la transfection ou la transduction de la cellule capable d'activation ou de la cellule au moins partiellement activée avec des acides nucléiques, dans lequel les acides nucléiques sont conçus pour conférer un phénotype souhaité aux cellules.

13. Procédé selon l'une quelconque des revendications 1-11, comprenant en outre la transfection ou la transduction de la cellule capable d'activation ou de la cellule au moins partiellement activée avec des acides nucléiques, dans lequel la transfection ou la transduction avec les acides nucléiques génère des cellules exprimant une molécule chimérique.

14. Procédé selon la revendication 12 ou 13, dans lequel la cellule capable d'activation est une cellule T.

15. Procédé selon l'une quelconque des revendications 1-14, dans lequel le diamètre du complexe formé entre la cellule capable d'activation et un ou plusieurs supports est au moins 20 % plus grand que celui des cellules non complexées.
